# EUROPEAN PATENT SPECIFICATION

(11) **EP 3 086 808 B1**
(45) Date of publication and mention of the grant of the patent: **19.09.2018**
(21) Application number: 14838861.4
(22) Date of filing: 24.12.2014
(51) Int. Cl.: A61K 39/395, C07K 16/28, C07K 16/40, A61K 39/00, A61P 35/00

(54) **ANTI ADAM17 ANTIBODY AND ITS USE FOR THE TREATMENT OF CANCER**
ANTI-ADAM17-ANTIKÖRPER UND IHRE VERWENDUNG BEI DER KREBSBEHANDLUNG
ANTICORPS ANTI ADAM17 ET LEUR UTILISATION DANS LE TRAITEMENT DU CANCER

(30) Priority: 24.12.2013 EP 13306860
(43) Date of publication of application: 02.11.2016
(73) Proprietor: Pierre Fabre Médicament, 92100 Boulogne-Billancourt (FR)
(72) Inventor: LOWE, Peter, F-01300 Chazey Bons (FR)
(74) Representative: Regimbeau
(86) International application number: PCT/EP2014/079315
(87) International publication number: WO 2015/097287

(56) References cited:
- WO-A1-2012/104581
- INKEN LORENZEN ET AL: "The membrane-proximal domain of A Disintegrin and Metalloprotease 17 (ADAM17) is responsible for recognition of the interleukin-6 receptor and interleukin-1 receptor II", FEBS LETTERS, ELSEVIER, AMSTERDAM, NL, vol. 586, no. 8, 8 March 2012 (2012-03-08) , pages 1093-1100, XP028412396, ISSN: 0014-5793, DOI: 10.1016/J.FEBSLET.2012.03.012 [retrieved on 2012-03-21]
- AHMAD TRAD ET AL: "ADAM17-overexpressing breast cancer cells selectively targeted by antibody-toxin conjugates", CANCER IMMUNOLOGY, IMMUNOTHERAPY, vol. 62, no. 3, 1 September 2012 (2012-09-01), pages 411-421, XP055112460, ISSN: 0340-7004, DOI: 10.1007/s00262-012-1346-x
- KOSUKE YAMAMOTO ET AL: "A novel bispecific single-chain antibody for ADAM17 and CD3 induces T-cell-mediated lysis of prostate cancer cells", BIOCHEMICAL JOURNAL, vol. 402, no. 1, 15 June 2012 (2012-06-15) , pages 884-144, XP055112455, ISSN: 0264-6021, DOI: 10.1016/j.ccr.2012.01.017
- AHMAD TRAD ET AL: "Development of sandwich ELISA for detection and quantification of human and murine a disintegrin and metalloproteinase17", JOURNAL OF IMMUNOLOGICAL METHODS, ELSEVIER SCIENCE PUBLISHERS B.V.,AMSTERDAM, NL, vol. 371, no. 1, 16 June 2011 (2011-06-16) , pages 91-96, XP028254105, ISSN: 0022-1759, DOI: 10.1016/J.JIM.2011.06.015 [retrieved on 2011-06-24]
- FRANCES M. RICHARDS ET AL: "Anti-Tumour Effects of a Specific Anti-ADAM17 Antibody in an Ovarian Cancer Model In Vivo", PLOS ONE, vol. 7, no. 7, 11 July 2012 (2012-07-11), page e40597, XP055112457, DOI: 10.1371/journal.pone.0040597
- CHRISTOPHER J TAPE ET AL: "Cross-domain inhibition of TACE ectodomain", NATIONAL ACADEMY OF SCIENCES. PROCEEDINGS, NATIONAL ACADEMY OF SCIENCES, UNITED STATES, vol. 108, no. 14, 5 April 2011 (2011-04-05), pages 5578-5583, XP002675381, ISSN: 1091-6490, DOI: 10.1073/PNAS.1017067108 [retrieved on 2011-03-17]
- "Recombinant Human TACE/ADAM17", , 11 June 2013 (2013-06-11), XP055112521, Retrieved from the Internet: URL:http://www.rndsystems.com/pdf/930-adb. pdf [retrieved on 2014-04-07]

## Description

A novel antibody capable of binding to ADAM17 is herein provided, as well as the amino and nucleic acid sequences coding for said antibody. From one aspect, it is described herein a novel antibody, or antigen-binding fragments, capable of binding to ADAM17 with anti-tumour activities. The use of said antibody as a drug for the treatment of cancer is also described. Finally, compositions comprising said antibody, alone or in combination or conjugation with other anti-cancer compounds, and the use of same for the treatment of cancer, are provided.

ADAM17 (A disintegrin and metalloproteinase domain-containing protein 17) also referred to as Snake venom-like protease, TNF-alpha convertase, TNF-alpha-converting enzyme (TACE) and CD156b is a membrane bound metalloprotease responsible for the extracellular cleavage (ectodomain shedding) of a number of pathologically important substrates. Originally identified as the enzyme responsible for the cleavage of membrane bound pro-TNF-□ liberating soluble protein (Black R.A. et al., 1997 (Nature. 1997; 385(6618):729-33), Moss M.L. et al., 1997 (Nature. 1997; 385(6618):733-6)), ADAM17 has since been described in the ectodomain shedding of a large number of membrane bound precursors protein (Black R.A. et al., 1997 (Nature. 1997; 385(6618):729-33), Moss M.L. et al., 1997 (Nature. 1997; 385(6618):733-6)). Ectodomain shedding by ADAM17 releases from the membrane of cells a large number of soluble cytokines and growth factors such as: Amphiregulin, Heparin binding-EGF like growth factor (HB-EGF), Transforming growth factor alpha (TGF-α, epiregulin, epigen and neuregulins (Arribas J. et al., (Curr Pharm Des. 2009; 15(20):2319-35.)). ADAM17 also mediates the shedding of numerous receptors including; IL-6R□, IL-1RII, Her4, c-Kit, Notch, Mer, TNF-α RI & II (Arribas, 2009) where the physiological result can be signal silencing through receptor shedding or soluble ligand trapping, or receptor transactivation as is described for IL-6R□□and gp130 (Chalaris A. et al., 2011 (Eur J Cell Biol. 2011; 90(6-7):484-94.)). ADAM17 can actively participate in the remodelling of the extracellular matrix and cell-cell contacts through the shedding of a large number of adhesion molecules and constituents of the extracellular microenvironment such as: L-selectin, ICAM-1, VCAM-1, Nectin-4, CD44 and collagen XVII. Less well understood activities of ADAM17 include the ectodomain shedding of cellular prion protein and amyloid precursor protein.

For the avoidance of doubt, without any specification, the expression ADAM17 refers to the human ADAM17 of sequence SEQ ID No. 29.

Structurally, ADAM17 consists of an 824 amino acids protein comprising a preproprotein domain (aa 1-214), an extracellular domain (aa 215-671), a transmembrane domain (aa 672-692) and a cytoplasmic domain (aa 693-824).

More particularly, the extracellular domain is comprised of a Metalloprotease (MP) domain of sequence SEQ ID No. 30 (corresponding to aa 215-474 of ADAM17), a Disintegrin (DI) domain of sequence SEQ ID No. 31 (corresponding to aa 475-563 of ADAM17) and a Membrane proximal (MPD) domain of sequence SEQ ID No. 32 (corresponding to aa 564-671 of ADAM17).

ADAM17 has been described as a technically difficult target for the generation of antagonistic antibodies indeed complex selection strategies have been described that necessitate selection and optimisation of specific binders to generate the desired characteristics. To date only one such specific antagonist has been described: the antibody D1(A12) capable of binding to ADAM17 through the disintegrin-cysteine rich and catalytic domains simultaneously. The antagonistic activity of D1(A12) is dependent on binding both the disintegrin-cysteine rich and catalytic domain to demonstrate its antagonistic activity.

The present invention intends to remedy the lack of relevant antibodies targeting ADAM17 with an efficient anti-tumoural activity.

In a first aspect, it is herein provided an antibody, or an antigen-binding fragment thereof, capable of binding to ADAM17.

In a particular aspect, the antibody herein described is capable of binding to ADAM17 through a single domain, contrary to the antibody described in the prior art. In other words, the present antibody is capable of binding to a single epitope whereas antibodies of the prior art bind to a conformational epitope composed of two different separated domains, the disintegrin-cysteine rich domain and the catalytic domain.

This property is of particular interest as the binding of the antibody is not dependent to a particular conformation of ADAM17 according to the nature of the patient, the nature of the condition to be treated, the general state of the patient, etc.

In an embodiment, it is described a monoclonal antibody, or an antigen-binding fragment thereof, that binds to an ADAM17 epitope and that inhibits the shedding of at least one substrate of ADAM17 wherein the said ADAM17 epitope consists of a single epitope comprised within the membrane proximal domain (MPD) of sequence SEQ ID No. 32.

In an embodiment, it is described a monoclonal antibody, or an antigen-binding fragment thereof, that binds to an ADAM17 epitope and that inhibits the shedding of at least one substrate of ADAM17 wherein the said ADAM17 epitope consists of an epitope comprised within the membrane proximal domain (MPD) of sequence SEQ ID No. 32.

An antibody binding to the MPD of ADAM17 can be obtained by any of a number of techniques well known to those skilled in the art, including but not limited to, immunisation and hybridoma generation, monoclonal B-cell selection, phage display, ribosomal display, yeast display, expressed immune response sequencing coupled with targeted gene synthesis. Each process can be performed with the ADAM17 protein or a selected sub domain as the target antigen. One skilled in the art could select for MPD binding antibodies from a population of antibodies binding to the ADAM17 extracellular domain, or more particularly the MPD. Subsequent selection and characterisation may in also represent the selective step for MPD binding whereby all binders to the ADAM17 extracellular domain are selectively screened for binding to the MPD or selected sub domains of the MPD. Alternatively all selection steps may be performed against the MPD or selected sub domains of the MPD and binding to the native ADAM17 extracellular domain being employed as a subsequent selection and characterisation step.

According to a particular embodiment, the antibody, or an antigen-binding fragment thereof, is characterized in that it consists of a monoclonal antibody.

"Monoclonal antibody" is understood to mean an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen.

By "binding", "binds", or the like, it is intended that the antibody, or antigen binding fragment thereof, forms a complex with an antigen that is relatively stable under physiologic conditions. Methods for determining whether two molecules bind are well known in the art and include, for example, equilibrium dialysis, surface plasmon resonance, and the like. For the avoidance of doubt, it does not mean that the said antibody or antigen-binding fragment could not bind or interfere, at a low level, to another antigen. As a preferred embodiment, the said antibody, or antigen-binding fragment thereof, binds to its antigen with an affinity that is at least two-fold greater than its affinity for binding to a non-specific molecule (BSA, casein, etc.). Nevertheless, as another preferred embodiment, the said antibody, or antigen-binding fragment thereof, binds only to the said antigen.

The Membrane proximal domain (MPD), as already mentioned, consists of the amino acid domain 564-671 of the human ADAM17 (of sequence SEQ ID No. 29). It can also be noticed here that the corresponding MPD for the murine ADAM17 is also comprised of the amino acids 564-671.

According to a particular aspect, the antibody herein described does not bind to the Metalloprotease (MP) domain, said MP domain being comprised of amino acid 215-474 of sequence SEQ ID No. 29.

According to a particular aspect, the antibody herein described does not bind to the Disintegrin (DI) domain, said DI domain being comprised of amino acid 475-563 of sequence SEQ ID No. 29.

This aspect is surprising as it has never been described, nor suggested, an antagonist, and more particularly an antibody, capable of decreasing or inhibiting the shedding of ADAM17 substrates without interfering with the catalytic domain of ADAM17, known to be responsible for the shedding activity. In other words, the antibody herein described is capable of selectively decreasing or inhibiting the enzymatic activity of ADAM17 regarding at least one substrate in the specific context of a pathology, said pathology being cancer. An advantage of the antibody herein described may rely on the fact that it seems to not inhibit the whole catalytic activity of ADAM17 as it does not bind to the catalytic domain, but it may be capable of decreasing or inhibiting the enzymatic activity of ADAM17 for all or part of its substrates.

In an embodiment, the antibody described in the present application inhibits the shedding of at least one substrate of ADAM17 with an IC50 of 500 pM or less, preferentially 200 pM or less and more preferentially 100 pM or less.

In the present context, the expression "IC50" refers to the concentration of an antibody in a dose response evaluation that is necessary to achieve half the maximal attainable inhibition. Such evaluation of the IC50 can be made by substrate shedding from cells evaluation or Fluorescence Resonance Energy Transfer (FRET) peptide cleavage assay with recombinant protein.

In a preferred aspect, the present antibody is capable of inhibiting the shedding of TNF-α (Tumor necrosis factor alpha), and more preferably with at least an IC50 of 500 pM or less, preferentially 200 pM or less and more preferentially 100 pM or less.

In a preferred aspect, the present antibody is capable of inhibiting the shedding of TGF-α (Transforming growth factor alpha), and more preferably with at least an IC50 of 500 pM or les, preferentially 200 pM or less and more preferentially 100 pM or less.

In a preferred aspect, the present antibody is capable of inhibiting the shedding of amphiregulin (AREG), and more preferably with at least an IC50 of 500 pM or less, preferentially 200 pM or less and more preferentially 100 pM or less.

In a preferred aspect, the present antibody is capable of inhibiting the shedding of HB-EGF (Heparin-binding EGF-like growth factor), and more preferably with at least an IC50 of 500 pM or les, preferentially 200 pM or less and more preferentially 100 pM or less.

In an embodiment, the present antibody is characterized in that it inhibits the shedding of at least one substrate of ADAM17 selected from TNF-α, TGF-α, AREG and HB-EGF.

In an embodiment, the present antibody is characterized in that it inhibits with at least an IC50 of 500 pM or less, preferentially 200 pM or less and more preferentially 100 pM or less, the shedding of at least one substrate of ADAM17 selected from TNF-α, TGF-α, AREG and HB-EGF.

In an embodiment, the present antibody is characterized in that it inhibits with at least an IC50 of 500 pM or less, preferentially 200 pM or less and more preferentially 100 pM or less, the shedding of TNF-α, TGF-α, AREG and HB-EGF.

According to another aspect, the antibody is characterized in that it binds to an ADAM17 epitope with a Kd of about 10 nM or less, preferentially of about 5 nM or less, as determined by surface plasmon resonance (SPR).

In another embodiment, the antibody is characterized in that it binds to an ADAM17 epitope with a Kd of about 10 nM or less, preferentially of about 5 nM or less, and more preferentially of about 2 nM or less, as determined by surface plasmon resonance (SPR).

"Kd" also referred to as "Kd" or "KD" refers to the dissociation constant of a particular antibody-antigen complex. Kd = Koff / Kon with Koff consisting in the off-rate constant for dissociation of the antibody from an antibody-antigen complex and Kon consisting in the rate at which the antibody associates with the antigen (Chen Y. et al., 1999; J Mol Biol. 1999;293(4):865-81)).

It must be understood here that the antibody disclosed herein is not in natural form, that is to say it is not in its natural environment but that it has been able to be isolated or obtained by purification from natural sources, or else obtained by genetic recombination, or by chemical synthesis, and that it can then contain unnatural amino acids as will be described herein.

Provided herein is an antibody, or an antigen-binding fragment thereof, capable of binding to an ADAM17 epitope and comprising six CDRs (complementarity-determining region), wherein at least one, preferentially at least two, preferentially at least three, preferentially at least four and more preferentially at least five of the six CDRs are selected from the CDRs comprising the amino acid sequences SEQ ID Nos. 1 to 6 or any sequences having at least 80%, preferably 85%, 90%, 95% and 98% identity after optimal alignment with sequences SEQ ID Nos. 1 to 6.

In another embodiment, the present antibody, or any antigen-binding fragment thereof, comprises the six CDRs of sequences SEQ ID Nos. 1 to 6, or any sequence having at least 80%, preferably 85%, 90%, 95% and 98% identity after optimal alignment with sequences SEQ ID Nos. 1 to 6.

Another embodiment relates to a monoclonal antibody, or an antigen-binding fragment thereof, which binds to an ADAM17 epitope, said antibody comprising:
- a heavy chain comprising CDR-H1, CDR-H2 and CDR-H3 of amino acid sequences SEQ ID Nos. 1, 2 and 3, respectively; and
- a light chain comprising CDR-L1, CDR-L2 and CDR-L3 of amino acid sequences SEQ ID Nos. 4, 5 and 6, respectively.

In an embodiment, the CDR-H1 comprises the sequence SEQ ID No.1 wherein the residue referred to as X₁ is selected from polar amino-acids. The polar amino-acid is preferentially selected from asparagine (Asn or N), aspartic acid (Asp or D), glutamine (Gln or Q), serine (Ser or S), glutamic acid (Glu or E), arginine (Arg or R), lysine (Lys or K), histidine (His or H), tryptophan (Trp or W), tyrosine (Tyr or Y) or threonine (Thr or T).

In another preferred embodiment, the residue X₁ is selected from the small size polar amino-acid. The small size polar amino-acid is preferentially selected from asparagine (Asn or N), aspartic acid (Asp or D), serine (Ser or S) or threonine (Thr or T).

In another preferred embodiment, the residue X₁ is asparagine (Asn or N).

The antibody subject of the present application is characterized in that the CDR-H1 is of amino acid sequence SEQ ID No. 7.

In another preferred embodiment, the residue X₁ is aspartic acid (Asp or D).

The antibody subject of the present application is characterized in that the CDR-H1 is of amino acid sequence SEQ ID No. 8.

By the expression "antigen binding fragment" of an antibody, it is intended to indicate any peptide, polypeptide, or protein retaining the ability to bind to the target (also generally referred to as antigen) of the said antibody, generally the same epitope, and comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, or at least 200 contiguous amino acid residues, of the amino acid sequence of the antibody.

In a preferred embodiment, the said antigen binding fragment comprises at least one CDR of the antibody from which it is derived. Still in a preferred embodiment, the said antigen binding fragment comprises 2, 3, 4 or 5 CDRs, more preferably the 6 CDRs of the antibody from which it is derived.

The "antigen binding fragments" can be selected, without limitation, in the group consisting of Fv, scFv (sc for single chain), Fab, F(ab')₂, Fab', scFv-Fc fragments or diabodies, or fusion proteins with disordered peptides such as XTEN (extended recombinant polypeptide) or PAS motifs, or any fragment of which the half-life time would be increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation in a liposome, said fragments having at least one of the characteristic CDRs of the antibody. Preferably, said "antigen binding fragments" will be constituted or will comprise a partial sequence of the heavy or light variable chain of the antibody from which they are derived, said partial sequence being sufficient to retain the same specificity of binding as the antibody from which it is descended and a sufficient affinity, preferably at least equal to 1/100, in a more preferred manner to at least 1/10, of the affinity of the antibody from which it is descended, with respect to the target.

The term "epitope" is a region of an antigen that is bound by an antibody. Epitopes may be defined as structural or functional. Functional epitopes are generally a subset of the structural epitopes and have those residues that directly contribute to the affinity of the interaction. Epitopes may also be conformational. In certain embodiments, epitopes may include determinants that are chemically active surface groupings of molecules such as amino acids, sugar side chains, phosphoryl groups, or sulfonyl groups, and, in certain embodiments, may have specific three-dimensional structural characteristics, and/or specific charge characteristics.

For the avoidance of doubt, the expression "single epitope" does not necessarily mean a linear epitope.

For the avoidance of doubt, without any contrary indication in the text, the expression CDRs means the hypervariable regions of the heavy and light chains of an antibody as defined by IMGT.

The IMGT unique numbering has been defined to compare the variable domains whatever the antigen receptor, the chain type, or the species [Lefranc M.-P., Immunology Today 18, 509 (1997) / Lefranc M.-P., The Immunologist, 7, 132-136 (1999) / Lefranc, M.-P., Pommié, C., Ruiz, M., Giudicelli, V., Foulquier, E., Truong, L., Thouvenin-Contet, V. and Lefranc, Dev. Comp. Immunol., 27, 55-77 (2003)]. In the IMGT unique numbering, the conserved amino acids always have the same position, for instance cystein 23 (1st-CYS), tryptophan 41 (CONSERVED-TRP), hydrophobic amino acid 89, cystein 104 (2nd-CYS), phenylalanine or tryptophan 118 (J-PHE or J-TRP). The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. As gaps represent unoccupied positions, the CDR-IMGT lengths (shown between brackets and separated by dots, e.g. [8.8.13]) become crucial information. The IMGT unique numbering is used in 2D graphical representations, designated as IMGT Colliers de Perles [Ruiz, M. and Lefranc, M.-P., Immunogenetics, 53, 857-883 (2002) / Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., T cell receptor and MHC structural data. Nucl. Acids. Res., 32, D208-D210 (2004)].

Three heavy chain CDRs and 3 light chain CDRs exist. The term CDR or CDRs is used here in order to indicate, according to the case, one of these regions or several, or even the whole, of these regions which contain the majority of the amino acid residues responsible for the binding by affinity of the antibody for the antigen or the epitope which it recognizes.

The present antibody, or any antigen binding fragment thereof can also be described as comprising: i) a heavy chain comprising CDR-H1, CDR-H2 and CDR-H3 comprising respectively amino acid sequences SEQ ID Nos. 1, 2 and 3, or sequences with at least 80%, preferably 85%, 90%, 95% and 98% identity after optimal alignment with sequences SEQ ID Nos. 1, 2 and 3; and ii) a light chain comprising CDR-L1, CDR-L2 and CDR-L3 comprising respectively amino acid sequences SEQ ID Nos. 4, 5 and 6, or sequences with at least 80%, preferably 85%, 90%, 95% and 98% identity after optimal alignment with sequences SEQ ID Nos. 4, 5 and 6.

As used herein, the "percentage identity" or "% identity" between two sequences of nucleic acids or amino acids means the percentage of identical nucleotides or amino acid residues between the two sequences to be compared, obtained after optimal alignment, this percentage being purely statistical and the differences between the two sequences being distributed randomly along their length. The comparison of two nucleic acid or amino acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981) [Ad. App. Math. 2:482], by means of the local homology algorithm of Neddleman and Wunsch (1970) [J. Mol. Biol. 48:443], by means of the similarity search method of Pearson and Lipman (1988) [Proc. Natl. Acad. Sci. USA 85:2444] or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

The percentage identity between two nucleic acid or amino acid sequences is determined by comparing the two optimally-aligned sequences in which the nucleic acid or amino acid sequence to compare can have additions or deletions compared to the reference sequence for optimal alignment between the two sequences. Percentage identity is calculated by determining the number of positions at which the amino acid, nucleotide or residue is identical between the two sequences, preferably between the two complete sequences, dividing the number of identical positions by the total number of positions in the alignment window and multiplying the result by 100 to obtain the percentage identity between the two sequences.

For example, the BLAST program, "BLAST 2 sequences" (Tatusova et al., "Blast 2 sequences - a new tool for comparing protein and nucleotide sequences", FEMS Microbiol., 1999, Lett. 174:247-250) available on the site http://www.ncbi.nlm.nih.gov/gorf/bl2.html, can be used with the default parameters (notably for the parameters "open gap penalty": 5, and "extension gap penalty": 2; the selected matrix being for example the "BLOSUM 62" matrix proposed by the program); the percentage identity between the two sequences to compare is calculated directly by the program.

For the amino acid sequence exhibiting at least 80%, preferably 85%, 90%, 95% and 98% identity with a reference amino acid sequence, preferred examples include those containing the reference sequence, certain modifications, notably a deletion, addition or substitution of at least one amino acid, truncation or extension. In the case of substitution of one or more consecutive or non-consecutive amino acids, substitutions are preferred in which the substituted amino acids are replaced by "equivalent" amino acids. Here, the expression "equivalent amino acids" is meant to indicate any amino acids likely to be substituted for one of the structural amino acids without however modifying the biological activities of the corresponding antibodies and of those specific examples defined below.

Equivalent amino acids can be determined either on their structural homology with the amino acids for which they are substituted or on the results of comparative tests of biological activity between the various antibodies likely to be generated.

As a non-limiting example, table 1 below summarizes the possible substitutions likely to be carried out without resulting in a significant modification of the biological activity of the corresponding modified antibody; inverse substitutions are naturally possible under the same conditions.

**Table 1**

| **Original residue** | **Substitution(s)** |
|---|---|
| Ala (A) | Val, Gly, Pro, Ser, Thr |
| Arg (R) | Lys, His, Gln |
| Asn (N) | Gln, Asp, His, Lys, Ser, Thr |
| Asp (D) | Glu, Asn |
| Cys (C) | Ser |
| Gln (Q) | Asn, Arg, Glu, His, Lys, Met |
| Glu (G) | Asp, Gln, Lys |
| Gly (G) | Ala, Pro |
| His (H) | Arg, Asn, Gln, Tyr |
| Ile (I) | Leu, Val, Met |
| Leu (L) | Ile, Val, Met, Phe |
| Lys (K) | Arg, Gln, Glu, Asn |
| Met (M) | Leu, Ile, , Gln, Val |
| Phe (F) | Tyr, Met, Leu, Trp |
| Pro (P) | Ala |
| Ser (S) | Thr, Cys, Ala, Asn |
| Thr (T) | Ser, Ala, Asn |
| Trp (W) | Tyr, Phe |
| Tyr (Y) | Phe, Trp, His |
| Val (V) | Leu, Ala, Ile, Met |

The present antibody, or any antigen binding fragment thereof can also be described as comprising: i) a heavy chain comprising CDR-H1, CDR-H2 and CDR-H3 comprising respectively amino acid sequences SEQ ID Nos. 7, 2 and 3, or sequences with at least 80%, preferably 85%, 90%, 95% and 98% identity after optimal alignment with sequences SEQ ID Nos. 7, 2 and 3; and ii) a light chain comprising CDR-L1, CDR-L2 and CDR-L3 comprising respectively amino acid sequences SEQ ID Nos. 4, 5 and 6, or sequences with at least 80%, preferably 85%, 90%, 95% and 98% identity after optimal alignment with sequences SEQ ID Nos. 4, 5 and 6.

The antibody, or any antigen binding fragment thereof, can also be described as comprising: i) a heavy chain comprising CDR-H1, CDR-H2 and CDR-H3 comprising respectively amino acid sequences SEQ ID Nos. 8, 2 and 3, or sequences with at least 80%, preferably 85%, 90%, 95% and 98% identity after optimal alignment with sequences SEQ ID Nos. 8, 2 and 3; and ii) a light chain comprising CDR-L1, CDR-L2 and CDR-L3 comprising respectively amino acid sequences SEQ ID Nos. 4, 5 and 6, or sequences with at least 80%, preferably 85%, 90%, 95% and 98% identity after optimal alignment with sequences SEQ ID Nos. 4, 5 and 6.

According to still another embodiment, the present antibody, or an antigen binding fragment thereof, comprises a heavy chain variable domain of sequence comprising the amino acid sequence SEQ ID No. 9 or a sequence with at least 80%, preferably 85%, 90%, 95% and 98% identity after optimal alignment with sequence SEQ ID No. 9.

According to still another embodiment, the present antibody, or an antigen binding fragment thereof, comprises a heavy chain variable domain of sequence comprising the amino acid sequence SEQ ID No. 11 or a sequence with at least 80%, preferably 85%, 90%, 95% and 98% identity after optimal alignment with sequence SEQ ID No. 11.

According to still another embodiment, the present antibody, or an antigen binding fragment thereof, comprises a heavy chain variable domain of sequence comprising the amino acid sequence SEQ ID No. 12 or a sequence with at least 80%, preferably 85%, 90%, 95% and 98% identity after optimal alignment with sequence SEQ ID No. 12.

In an embodiment, the antibody is characterized in that it comprises a heavy chain variable domain of sequence comprising the amino acid sequence SEQ ID No. 9, 11 or 12.

According to still another embodiment, the present antibody, or an antigen binding fragment thereof, comprises a light chain variable domain of sequence comprising the amino acid sequence SEQ ID No. 10 or a sequence with at least 80%, preferably 85%, 90%, 95% and 98% identity after optimal alignment with sequence SEQ ID No. 10.

In an embodiment, the antibody is characterized in that it comprises a light chain variable domain of sequence comprising the amino acid sequence SEQ ID No. 10.

According to still another embodiment, the present antibody, or an antigen binding fragment thereof, comprises
i) a heavy chain variable domain of sequence comprising the amino acid sequence selected from SEQ ID No. 9, 11 or 12 or a sequence with at least 80%, preferably 85%, 90%, 95% and 98% identity after optimal alignment with sequence SEQ ID No. 9, 11 or 12, and
ii) a light chain variable domain of sequence comprising the amino acid sequence SEQ ID No. 10 or a sequence with at least 80%, preferably 85%, 90%, 95% and 98% identity after optimal alignment with sequence SEQ ID No. 10.

More particularly, the present antibody comprises a heavy chain variable domain of sequence comprising the amino acid sequence SEQ ID No. 9, 11 or 12; and a light chain variable domain of sequence comprising the amino acid sequence SEQ ID No. 10.

In an embodiment, the antibody, or an antigen binding fragment thereof, comprises i) a heavy chain variable domain of sequence comprising the amino acid sequence SEQ ID No. 9, and ii) a light chain variable domain of sequence comprising the amino acid sequence SEQ ID No. 10.

In an embodiment, the antibody, or an antigen binding fragment thereof, comprises i) a heavy chain variable domain of sequence comprising the amino acid sequence SEQ ID No. 11, and ii) a light chain variable domain of sequence comprising the amino acid sequence SEQ ID No. 10.

In an embodiment, the antibody, or an antigen binding fragment thereof, comprises i) a heavy chain variable domain of sequence comprising the amino acid sequence SEQ ID No. 12, and ii) a light chain variable domain of sequence comprising the amino acid sequence SEQ ID No. 10.

In an embodiment, the antibody consists of a murine antibody. In this case, it is referred as m1022C3.

In an embodiment, the antibody consists of a chimeric antibody. In this case, it is referred as c1022C3.

In an embodiment, the antibody consists of a humanized antibody. In this case, it is referred as hz1022C3.

In an embodiment, the antibody consists of a human antibody. In this case, it is referred as h1022C3.

The expression 1022C3 can also be used to refer to the antibody irrespectively of its origin, meaning it encompasses m/c/h/hz 1022C3.

For more clarity, table 2 below summarizes the various amino acid sequences corresponding to the present antibody.

**Table 2**

| **Antibody** | **CDR numbering** | **Heavy chain** | **Light chain** | **SEQ ID NO.** |
|---|---|---|---|---|
| 1022C3 | IMGT | CDR-H1 (X₁) | | 1 |
| | | CDR-H1 (N) | | 7 |
| | | CDR-H1 (D) | | 8 |
| | | CDR-H2 | | 2 |
| | | CDR-H3 | | 3 |
| | | | CDR-L1 | 4 |
| | | | CDR-L2 | 5 |
| | | | CDR-L3 | 6 |
| | | variable domain (X₁) | | 9 |
| | | variable domain (N) | | 11 |
| | | variable domain (D) | | 12 |
| | | | variable domain | 10 |
| | | Chimeric (c) | | 33 |
| | | Chimeric (c) AlaAla | | 34 |
| | | | Chimeric (c) | 35 |

In another aspect, the antibody, or an antigen binding fragment thereof, consists of a chimeric antibody.

A chimeric antibody is one containing a natural variable region (light chain and heavy chain) derived from an antibody of a given species in combination with constant regions of the light chain and the heavy chain of an antibody of a species heterologous to said given species.

The antibodies, or chimeric fragments of same, can be prepared by using the techniques of recombinant genetics. For example, the chimeric antibody could be produced by cloning recombinant DNA containing a promoter and a sequence coding for the variable region of a nonhuman monoclonal antibody as described herein, notably murine, and a sequence coding for the human antibody constant region. A chimeric antibody coded by one such recombinant gene could be, for example, a mouse-human chimera, the specificity of this antibody being determined by the variable region derived from the murine DNA and its isotype determined by the constant region derived from human DNA. Refer to Verhoeyn et al. (BioEssays, 8:74, 1988) for methods for preparing chimeric antibodies.

A specific aspect relates to a chimeric antibody, or an antigen binding fragment thereof, comprising:
i) a heavy chain comprising CDR-H1, CDR-H2 and CDR-H3 comprising respectively amino acid sequences SEQ ID Nos. 1, 2 and 3, or sequences with at least 80%, preferably 85%, 90%, 95% and 98% identity after optimal alignment with sequences SEQ ID Nos. 1, 2 and 3;
ii) a light chain comprising CDR-L1, CDR-L2 and CDR-L3 comprising respectively amino acid sequences SEQ ID Nos. 4, 5 and 6, or sequences with at least 80%, preferably 85%, 90%, 95% and 98% identity after optimal alignment with sequences SEQ ID Nos. 4, 5 and 6; and
iii) said antibody also comprising light-chain and heavy-chain constant regions derived from an antibody of a species heterologous with the mouse.

In an embodiment, the said species heterologous with the mouse is human (also possibly referred to as man).

As an example, the chimeric antibody is characterized in that it comprises a heavy chain of sequence comprising the amino acid sequence SEQ ID No. 33; and/or a light chain of sequence comprising the amino acid sequence SEQ ID No. 35.

As an example, the chimeric antibody is characterized in that it comprises a heavy chain of sequence comprising the amino acid sequence SEQ ID No. 34; and, optionally, a light chain of sequence comprising the amino acid sequence SEQ ID No. 35.

In a more preferred embodiment, the chimeric antibody is characterized in that it comprises a heavy chain of sequence comprising the amino acid sequence SEQ ID No. 33 or 34 and a light chain of sequence comprising the amino acid sequence SEQ ID No. 35.

Another aspect relates to an antibody, or an antigen binding fragment thereof, which consists of a humanized antibody.

"Humanized antibody" means an antibody that contains CDR regions derived from an antibody of nonhuman origin, the other parts of the antibody molecule being derived from one (or several) human antibodies. In addition, some of the skeleton segment residues (called FR) can be modified to preserve binding affinity (Jones et al., Nature, 321:522-525, 1986; Verhoeyen et al., Science, 239:1534-1536, 1988; Riechmann et al., Nature, 332:323-327, 1988).

The humanized antibodies disclosed herein or fragments of same can be prepared by techniques known to a person skilled in the art (such as, for example, those described in the documents Singer et al., J. Immun., 150:2844-2857, 1992; Mountain et al., Biotechnol. Genet. Eng. Rev., 10:1-142, 1992; and Bebbington et al., Bio/Technology, 10:169-175, 1992). Such humanized antibodies are preferred for their use in methods involving *in vitro* diagnoses or preventive and/or therapeutic treatment *in vivo.* Other humanization techniques, also known to a person skilled in the art, such as, for example, the "CDR grafting" technique described by PDL in patents EP 0 451 261, EP 0 682 040, EP 0 939 127, EP 0 566 647 or US 5,530,101, US 6,180,370, US 5,585,089 and US 5,693,761. US patents 5,639,641 or 6,054,297, 5,886,152 and 5,877,293 can also be cited.

A specific aspect relates to a humanized antibody, or an antigen binding fragment thereof, comprising:
i) a heavy chain comprising CDR-H1, CDR-H2 and CDR-H3 comprising respectively amino acid sequences SEQ ID Nos. 1, 2 and 3, or sequences with at least 80%, preferably 85%, 90%, 95% and 98% identity after optimal alignment with sequences SEQ ID Nos. 1, 2 and 3;
ii) a light chain comprising CDR-L1, CDR-L2 and CDR-L3 comprising respectively amino acid sequences SEQ ID Nos. 4, 5 and 6, or sequences with at least 80%, preferably 85%, 90%, 95% and 98% identity after optimal alignment with sequences SEQ ID Nos. 4, 5 and 6; and
iii) said antibody also comprising constant regions of the light-chain and the heavy-chain derived from human antibody.

In an embodiment, it is described a humanized antibody, or an antigen binding fragment thereof, comprising i) a heavy chain comprising CDR-H1, CDR-H2 and CDR-H3 comprising respectively amino acid sequences SEQ ID Nos. 1, 2 and 3, ii) a light chain comprising CDR-L1, CDR-L2 and CDR-L3 comprising respectively amino acid sequences SEQ ID Nos. 4, 5 and 6; and iii) said antibody also comprising constant regions of the light-chain and the heavy-chain derived from a human antibody.

In an embodiment, it is described a humanized antibody, or an antigen binding fragment thereof, comprising i) a heavy chain comprising CDR-H1, CDR-H2 and CDR-H3 comprising respectively amino acid sequences SEQ ID Nos. 7, 2 and 3, ii) a light chain comprising CDR-L1, CDR-L2 and CDR-L3 comprising respectively amino acid sequences SEQ ID Nos. 4, 5 and 6; and iii) said antibody also comprising constant regions of the light-chain and the heavy-chain derived from a human antibody.

In an embodiment, it is described a humanized antibody, or an antigen binding fragment thereof, comprising i) a heavy chain comprising CDR-H1, CDR-H2 and CDR-H3 comprising respectively amino acid sequences SEQ ID Nos. 8, 2 and 3, ii) a light chain comprising CDR-L1, CDR-L2 and CDR-L3 comprising respectively amino acid sequences SEQ ID Nos. 4, 5 and 6; and iii) said antibody also comprising constant regions of the light-chain and the heavy-chain derived from a human antibody.

In a preferred manner, constant regions of the light-chain and the heavy-chain derived from a human antibody are, respectively, the lambda or kappa and the gamma-1, gamma-2 or gamma-4 region.

It is described a monoclonal antibody, or an antigen-binding fragment thereof, characterized in that it consists of the monoclonal antibody 1022C3 obtained from the hybridoma I-4686 deposited at the CNCM, Institut Pasteur, 25 Rue du Docteur Roux, 75725 Paris Cedex 15, France, on the 18 October 2012.

Another aspect relates to a murine hybridoma capable of secreting the present monoclonal antibody, notably the hybridoma of murine origin filed with the French collection for microorganism cultures (CNCM, Pasteur Institute, Paris, France) on October 18, 2012, under number I-4686. Said hybridoma was obtained by the fusion of Balb/C immunized mice splenocytes and cells of the myeloma Sp 2/O-Ag 14 lines.

In a particular embodiment, the antibody herein described is characterized in that it is capable of binding to a single epitope of ADAM17 comprised within the membrane proximal domain (MPD) of sequence SEQ ID No. 32.

Another preferred property of the antibody is that it inhibits the shedding of at least one substrate of ADAM17 with an IC₅₀ of 500 pM or less, preferentially 200 pM or less and more preferentially 100 pM or less, said at least one substrate of ADAM17 being preferentially selected from TNF-α, TGF-α, AREG and/or HB-EGF.

In another embodiment, the antibody is characterized in that it binds to an ADAM17 epitope with a Kd of about 10 nM or less, preferentially of about 5 nM or less, and more preferentially of about 2 nM or less, as determined by surface plasmon resonance (SPR).

Another aspect is a method of inhibiting the growth and/or the proliferation of tumour cells wherein said method comprises a step of administering, to a patient in need thereof, an effective amount of an antibody, or an antigen-binding fragment thereof, as above described.

In another embodiment, it is described an antibody for use in a method of inhibiting the growth and/or the proliferation of tumour cells, said tumour cells being selected from tumour cells expressing ADAM17.

The person skilled in the art would easily determine the expression level of ADAM17 by any known technique such as cytometry, immunohistochemistry, Antibody Binding Capacity (ABC), etc.

As a non-limitative example, the expression level can be determined by measuring by cytometry the Antibody Binding Capacity (ABC) of a labelled antibody to ADAM17.

In an embodiment, the tumour cell is considered as expressing ADAM17 with an ABC of at least 5000.

In another embodiment, the tumour cell is considered as expressing ADAM17 with an ABC of at least 10000.

Another aspect relates to the murine hybridoma I-4686 deposited at the CNCM, Institut Pasteur, France, on the 18 October 2012.

Another aspect relates to an isolated nucleic acid, characterized in that it is chosen from the following nucleic acids:
a) nucleic acid coding for an antibody, or an antigen binding fragment thereof, as above described;
b) nucleic acid comprising the sequences SEQ ID No. 13-28 or sequences exhibiting a percentage identity of at least 80%, preferably 85%, 90%, 95% and 98%, after optimal alignment with SEQ ID No. 13-28 and 36 to 38.

An isolated nucleic acid is herein provided, characterized in that it is chosen from the following nucleic acids:
a) a DNA or RNA, coding for an antibody, or an antigen-binding fragment thereof, as above described; and
b) a DNA comprising one of the sequences selected from the group consisting of SEQ ID No. 13-28 and 36 to 38, preferably comprising the 6 nucleic acids encoding the CDRs H1, H2, H3 and L1, L2, L3, or a nucleic acid encoding the heavy and/or light chain, preferably the anti-ADAM17antibody variable domain selected from the group consisting of SEQ ID No. 13-28 and 36 to 38.

Table 3 below summarizes the various nucleotide sequences concerning the present antibody.

**Table 3**

| **Antibody** | **CDR numbering** | **Heavy chain** | **Light chain** | **SEQ ID NO.** |
|---|---|---|---|---|
| 1022C3 | IMGT | CDR-H1 (X₂) | | 13 |
| | | CDR-H1 (aac) | | 19 |
| | | CDR-H1 (aat) | | 20 |
| | | CDR-H1 (gac) | | 21 |
| | | CDR-H1 (gat) | | 22 |
| | | CDR-H2 | | 14 |
| | | CDR-H3 | | 15 |
| | | | CDR-L1 | 16 |
| | | | CDR-L2 | 17 |
| | | | CDR-L3 | 18 |
| | | variable domain (X₂) | | 23 |
| | | variable domain (aac) | | 24 |
| | | variable domain (aat) | | 27 |
| | | variable domain (gac) | | 28 |
| | | variable domain (gat) | | 25 |
| | | | variable domain | 26 |
| | | Chimeric (c) | | 36 |
| | | Chimeric (c) AlaAla | | 37 |
| | | | Chimeric (c) | 38 |

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, defining a fragment or a region of a nucleic acid, containing unnatural nucleotides or not, and being either a double-strand DNA, a single-strand DNA or transcription products of said DNAs.

It should also be included here that the present disclosure does not relate to nucleotide sequences in their natural chromosomal environment, i.e., in a natural state. The present sequences have been isolated and/or purified, i.e., they were sampled directly or indirectly, for example by a copy, their environment having been at least partially modified. Isolated nucleic acids obtained by recombinant genetics, by means, for example, of host cells, or obtained by chemical synthesis should also be mentioned here.

"Nucleic sequences exhibiting a percentage identity of at least 80%, preferably 85%, 90%, 95% and 98%, after optimal alignment with a preferred sequence" means nucleic sequences exhibiting, with respect to the reference nucleic sequence, certain modifications such as, in particular, a deletion, a truncation, an extension, a chimeric fusion and/or a substitution, notably punctual. Preferably, these are sequences which code for the same amino acid sequences as the reference sequence, this being related to the degeneration of the genetic code, or complementarity sequences that are likely to hybridize specifically with the reference sequences, preferably under highly stringent conditions.

It is also described a vector comprising a nucleic acid as above mentioned.

Such vectors are notably cloning and/or expression vectors that contain such a nucleotide sequence.

The vector preferably contains elements which allow the transcription/translation expression and/or the secretion of nucleotide sequences in a given host cell, resulting in the expression/secretion of the encoded protein. The vector thus must contain a promoter, translation initiation and termination signals, as well as suitable transcription regulation regions. In a preferred embodiment, such vector should be able to be maintained in a stable manner in the host cell and may optionally have specific signals which specify secretion of the translated protein. These various elements are selected and optimized by a person skilled in the art according to the host cell used. For this purpose, the nucleotide sequences can be inserted in self-replicating vectors within the chosen host or be integrative vectors of the chosen host.

Such vectors are prepared by methods typically used by a person skilled in the art and the resulting clones can be introduced into a suitable host by standard methods such as lipofection, electroporation, heat shock or chemical methods.

The vectors are, for example, vectors of plasmid or viral origin. They are used to transform host cells in order to clone or express the present nucleotide sequences.

It is also provided herein a host cell comprising a vector as above described.

The host cell can be selected among prokaryotic or eukaryotic systems such as bacterial cells, for example, but also yeast cells or animal cells, notably mammal cells. Insect or plant cells can also be used.

Another topic of the present application consists of a transgenic animal with the exception of human comprising at least one cell transformed by an antibody as above described.

It is thus also described a process for production of an antibody, or an antigen-binding fragment thereof, according to the present specification, characterized in that it comprises the following stages:
a) the culture in a medium and appropriate culture conditions of a cell as above described; and
b) the recovery of said antibody, or antigen-binding fragment thereof, thus produced starting from the culture medium or said cultured cells.

The transformed cells are of use in methods for the preparation of recombinant polypeptides described above. Methods for the preparation of said polypeptide in recombinant form, characterized in that said methods use a vector and/or a cell transformed by a vector described above, are also provided. Preferably, a cell transformed by such a vector is cultured under conditions that allow the expression of the aforesaid polypeptide and recovery of said recombinant peptide.

As already mentioned, the host cell can be selected among prokaryotic or eukaryotic systems. In particular, it is possible to identify the nucleotide sequences that facilitate secretion in such a prokaryotic or eukaryotic system. A vector carrying such a sequence can thus be used advantageously for the production of recombinant proteins to be secreted. Indeed, the purification of these recombinant proteins of interest will be facilitated by the fact that they are present in the supernatant of the cellular culture rather than inside host cells.

The polypeptides described above can also be prepared by chemical synthesis. One such method of preparation is provided herein. A person skilled in the art knows methods for chemical synthesis, such as solid-phase techniques (see notably Steward et al., 1984, Solid phase peptides synthesis, Pierce Chem. Company, Rockford, 111, 2nd ed.) or partial solid-phase techniques, by condensation of fragments or by conventional synthesis in solution. Polypeptides obtained by chemical synthesis and capable of containing corresponding unnatural amino acids are also described herein.

The antibody, or an antigen binding-fragment thereof, likely to be obtained by said method is also provided herein.

It is herein provided the antibody described above, or an antigen-binding fragment thereof, for use as a drug.

It is also herein provided a composition characterized in that it comprises, in addition, another therapeutically effective compound consisting of an antitumour compound.

In an embodiment, the present application relates to a composition comprising the antibody as above described.

In an embodiment, the composition is characterized in that it further comprises, as a combination product, a therapeutically effective amount of a cytotoxic agent.

The constituents of which the combination is composed may be administered simultaneously, separately, or sequentially so as to obtain the maximum efficacy of the combination; it being possible for each administration to vary in its duration from a rapid administration to a continuous perfusion.

As used herein, "simultaneous administration" refers to the administration of the two compounds of the composition according in a single and unique pharmaceutical form.

As used herein, "separate administration" refers to the administration, at the same time, of the two compounds of said composition in distinct pharmaceutical forms.

As used herein, "sequential administration" refers to the successive administration of the two compounds of said composition, each in a distinct pharmaceutical form.

A "therapeutically effective amount", as used herein, refers to the minimum concentration or amount of a compound (or of compounds) which is effective to prevent, alleviate, reduce or ameliorate symptoms of disease or prolong the survival of the patient being treated. A therapeutically effective amount is also one in which any toxic or detrimental effects of the agent are outweighed by the therapeutically beneficial effects. More particularly, in reference to the treatment of cancer, a therapeutically effective amount refers to that amount which has the effect of (1) reducing the size of (or preferably eliminating) the tumour; (2) inhibiting (that is, slowing to some extent, preferably stopping) tumour metastasis; (3) inhibiting to some extent (that is slowing to some extent, preferably stopping) tumour growth; and/or, (4) relieving to some extent (or preferably eliminating) one or more symptoms associated with the cancer.

In an embodiment, the composition is characterized in that it further comprises, as a conjugation product, a therapeutically effective amount of a cytotoxic agent.

As used herein, the expression "conjugation" refers generally to a composition comprising at least the antibody above described physically linked with a one or more cytotoxic agent(s), thus creating a highly targeted compound.

In an embodiment, the antibody is chemically linked to the cytotoxic agent by a linker. "Linker", "Linker Unit", or "link" means a chemical moiety comprising a covalent bond or a chain of atoms that covalently attaches a binding protein to at least one cytotoxic agent. The linker may be a "non-cleavable" or "cleavable".

The composition may also contain various diluents, fillers, salts, buffers, stabilizers, solubilisers, and other materials well known in the art.

In another embodiment, the composition is a pharmaceutical composition further comprising a pharmaceutically acceptable vehicle.

As used herein, "pharmaceutically acceptable vehicle" or "pharmaceutically acceptable carrier" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of media and agents for pharmaceutically active substances is well known in the art. Methods for preparing parenterally administrable compounds will be known or apparent to those skilled in the art and are described in more detail in for example, Remington's Pharmaceutical Science, 17th ed., Mack Publishing Company, Easton, Pa. (1985), and the 18^{th} and 19^{th} editions thereof.

By "cytotoxic agent" or "cytotoxic", it is intended an agent which, when administered to a subject, treats or prevents the development of cell proliferation, preferably the development of cancer in the subject's body, by inhibiting or preventing a cellular function and/or causing cell death.

Many cytotoxic agents have been isolated or synthesized and make it possible to inhibit the cells proliferation, or to destroy or reduce, if not definitively, at least significantly the tumour cells.

More particularly, the cytotoxic agent may preferably consist of, without limitation, a drug, a toxin, a radioisotope, etc.

According to another aspect, it is described the composition for use in a method of inhibiting the growth and/or the proliferation of tumour cells.

The use of the composition for the preparation of a drug intended for the prevention or the treatment of cancer is also described herein.

Notably, the use of an antibody as above described, or obtained by the process above described, or of a composition above described for the treatment of cancer, said cancer being a cancer chosen from cancers expressing ADAM17, is described herein.

In a preferred embodiment, said cancer is a cancer chosen from ovarian cancer, breast cancer, kidney cancer, liver cancer, pancreatic cancer, lung cancer, head and neck cancer and epidermoid carcinoma.

For therapeutic applications, the present composition is administered to a mammal, preferably a human, in a pharmaceutically acceptable dosage form such as those discussed above, including those that may be administered to a human intravenously as a bolus or by continuous infusion over a period of time, by intramuscular, intraperitoneal, intracerebrospinal, subcutaneous, intra-articular, intrasynovial, intrathecal, oral, topical, or inhalation routes. Said composition is also suitably administered by intratumoural, peritumoural, intralesional, or perilesional routes, to exert local as well as systemic therapeutic effects.

### Legend of the Figures

Figure 1: FRET peptide cleavage assay
Figure 2: Effect of mAb 1022C3 on TGF□-Nluc release of A431-TGF□-Nluc cells.
Figure 3: Effect of mAb 1022C3 on AREG-Nluc release of A431-AREG-Nluc cells.
Figure 4: Effect of mAb 1022C3 on TNF□-Nluc release of A431-TNF□-Nluc cells.
Figure 5: Effect of mAb 1022C3 on HB-EGF-Nluc release of A431-HB-EGF-Nluc cells.
Figure 6: FRET peptide cleavage assay for 1022C3 variants.
Figure 7: Binding of m1022C3 glycosylated and enzymlatically deglycosylated to tumour cell line NCI-H1299.
Figure 8: mAb 1022C3 inhibition of AREG shedding on MCF7 cells.
Figure 9: Dose effect of mAb 1022C3 on AREG shedding on MCF7 cells.
Figure 10: In vivo activity of the 1022C3 on established A431 xenografts.
Figure 11: In vivo activity of the 1022C3 on a fragment A431 xenograft model.
Figure 12: Comparison of m1022C3 (murine form) and c1022C3 (chimeric form) on the A431 xenograft model.
Figure 13: In vivo activity of the 1022C3 on established CaOV-3 xenografts
Figure 14: In vivo activity of the 1022C3 on established OVISE xenografts.
Figure 15: In vivo activity if the 1022C3 on established BxPC3 xenografts.
Figure 16: In vivo activity of the 1022C3 on established BICR-22 xenografts.
Figure 17: In vivo activity of the 1022C3 on established JIMT-1 xenografts.
Figure 18: In vivo activity if the 1022C3 on established NCI-H292 xenografts.

### Example 1: Generation of the antibody

To generate murine monoclonal antibodies (mAbs) against human ADAM17, 5 BALB/c mice were immunized 3-times subcutaneously with 15-20 µg of the human ADAM17 recombinant protein (R and D Systems, ref: 930-ADB, rhADAM17). The first immunization was performed in the presence of Complete Freund's Adjuvant (Sigma, St Louis, MD, USA). Incomplete Freund's adjuvant (Sigma) was added for following immunizations.

Three days prior to the fusion, 2 immunized mice (selected based on sera titration) were boosted with 15-20 µg of rhADAM17 protein with incomplete Freund's adjuvant. Lymphocytes were prepared by mincing of the proximal lymph nodes, they were then fused to SP2/0-Ag14 myeloma cells in a 1:4 ratio (lymphocyte : myeloma) (ATCC, Rockville, MD, USA). The fusion protocol is that described by Kohler and Milstein (1975), finally, 50 96 well plates were seeded. Fused cells were then subjected to metabolic HAT selection. Approximately 10 days after the fusion, colonies of hybrid cells were screened. For the primary screen, supernatants of hybridomas were evaluated for the secretion of mAbs raised against human ADAM17 using an ELISA.

Briefly, 96-well ELISA plates (Costar 3690, Corning, NY, USA) were coated with 50 µl/well of the recombinant human ADAM17 protein (R and D Systems, ref: 930 ADB) at 0.7 µg/ml in PBS overnight at 4°C. The plates were then blocked with PBS containing 0.5% gelatin (#22151, Serva Electrophoresis GmbH, Heidelberg, Germany) for 2 h at 37°C. Once the saturation buffer discarded by flicking plates, 50 µl of sample (hybridoma supernatant or purified antibody) was added to the ELISA plates and incubated for 1 h at 37°C. After three washes, 50 µl horseradish peroxidase-conjugated polyclonal goat anti-mouse IgG (#115-035-164, Jackson Immuno-Research Laboratories, Inc., West Grove, PA, USA) was added at a 1/5000 dilution in PBS containing 0.1% gelatin and 0.05% Tween 20 (w:w) for 1 h at 37°C. ELISA plates were washed 3-times and TMB (#UP664782, Uptima, Interchim, France) substrate was added. After a 10-min incubation time at room temperature, the reaction was stopped using 1 M sulfuric acid and the optical density at 450 nm was measured.

As a second screening step, selected hybridoma supernatants were evaluated by FACS analysis for mAbs able to bind the cellular form of ADAM17 expressed on the surface of A172 human tumour cells. For the selection by flow cytometry, 2x10⁵ cells were plated in each well of a 96 well-plate in PBS containing 1% BSA and 0.01% sodium azide (FACS buffer) at 4°C. After a 2-min centrifugation at 2000 rpm, the buffer was removed and hybridoma supernatants to be tested were added. After 20 min of incubation at 4°C, cells were washed twice and an Alexa 488-conjugated goat anti-mouse antibody diluted 1/500 in FACS buffer (#A11017, Molecular Probes Inc., Eugene, USA) was added and incubated for 20 min at 4°C. After a final wash with FACS buffer, cells were analysed by FACS (Facscalibur, Becton-Dickinson) after addition of propidium iodide to each tube at a final concentration of 40 µg/ml. Wells containing cells alone and cells incubated with the secondary Alexa 488-conjugated antibody were included as negative controls. Isotype controls were used in each experiment (Sigma, ref M90351MG). At least 5000 cells were assessed to calculate the mean value of fluorescence intensity (MFI).

As soon as possible, selected hybridomas were cloned by limiting dilution. One 96-well plate was prepared for each code. A volume of 100 µl of a cell suspension adjusted to 8 cells/ml in cloning specific culture medium was loaded in each well. At Day 7, the wells were microscopically examined to ensure cloning and plating efficiency before refeeding the plates with 100 µl of cloning specific culture medium. At days 9-10, the hybridoma supernatants were subsequently screened for their reactivity against the rhADAM17 protein. Cloned mAbs were then isotyped using an Isotyping kit (cat #5300.05, Southern Biotech, Birmingham, AL, USA). One clone obtained from each hybridoma was selected and expanded to confirm their binding specificity against rhADAM17 and human tumour cells (A172).

### Example 2: FRET Peptide cleavage assay

In a black 96-well plate, 40 µl/well of recombinant human ADAM17 (R&D Systems, ref: 930 ADB) (250 ng/ml) was incubated with 10µl of the anti-ADAM17 mAb (m1022C3) or the irrelevant mAb (9G4) at different concentrations for 10 min at room temperature. Then, 50 µl of a 10µM solution of substrate peptide corresponding to pro-TNF□ ADAM17 specific cleaving site labelled at each end with 5-FAM and TAMRA (5-FAM-SPLAQAVRSSSRK-TAMRA) was added. Fluorescence was then measured successively after various 37 °C incubation times (t= 0, 2, 6 and 24 hours) on a Berthold Mithras plate reader (excitation filter 485 nm, emission filter 530 nm, energy lamp: 7000 reading time: 0.2 s/well). Fluorescence increase reflected ADAM17 activity since fluorescence of 5-FAM is quenched by TAMRA on uncleaved substrate peptide.

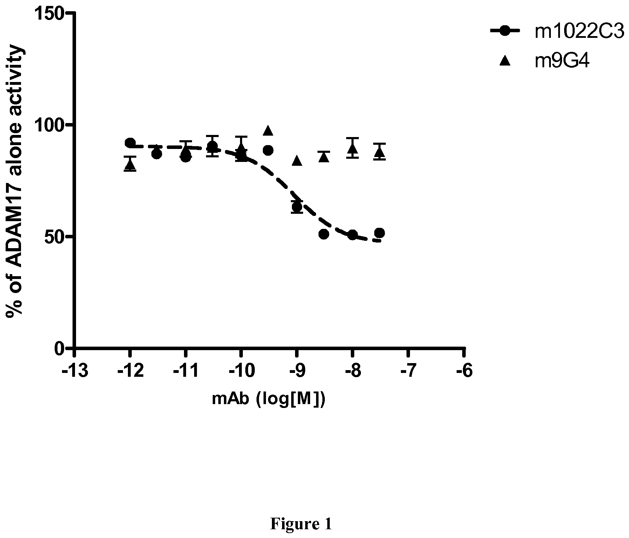

Anti ADAM17 mAb m1022C3 showed a dose-dependent inhibition of substrate peptide cleavage (Figure 1).

### Example 3: ADAM 17 shedding of recombinant substrates from tumour cell line A431

Stably transfected A431 cell lines, expressing at their plasma membrane pro-TGF□, pro-HB-EGF, pro-amphiregulin or a mutated pro-TNF□ each fused to NanoLuciferase® (Promega), were generated. ADAM17 activity at the plasma membrane of these cells resulted in the release in the culture medium of the mature substrates fused to NanoLuciferase®. Time dependant measurements of NanoLuciferase activity in culture medium samples reflected ADAM17 activity. A431 substrate-Nluc cells were seeded at 30 000 cells/well in a 96 wells culture plate. Two days later, culture medium was removed and replaced by 200 µl of fresh culture medium in which were diluted different concentrations of the anti-ADAM17 mAb (m1022C3) or the irrelevant mAb (9G4). After 24h of culture (37 °C, CO₂ 5%) 5 µl of culture medium from all experimental wells was collected and distributed in wells of white half-area 96 well plates. After addition of 15 µl of (PBS diluted) Nano-Glo™ luciferase substrate (furimazine), total luminescence for each experimental was read during 0.1s on a Berthold Mithras LB940 multimode microplate reader.

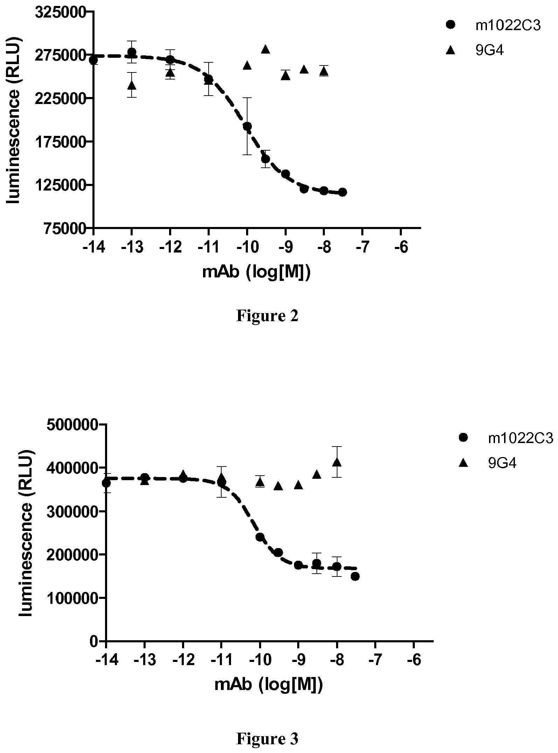

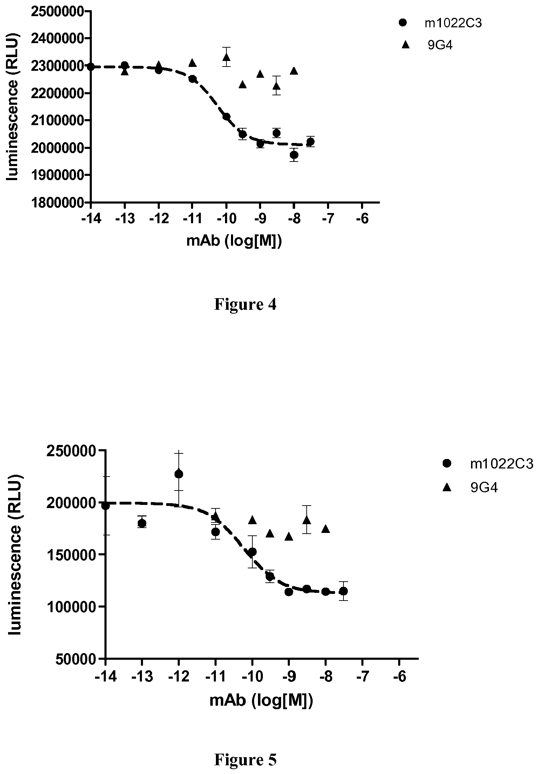

Anti ADAM17 mAb m1022C3 induced a dose-dependent decrease of i) TGF□-Nluc release in culture medium (figure 2), ii) AREG-Nluc release in culture medium (figure 3), iii) TNF□-Nluc release in culture medium (figure 4) and iv) HB-EGF-Nluc release in culture medium (figure 5). The IC₅₀ values obtained for the inhibition of shedding of TGF□, pro-HB-EGF, pro-amphiregulin or a mutated pro-TNF□□with mAb m1022C3 were compare to those published in the literature (Table 4). The mAb m1022C3 demonstrated significantly greater than 10-fold, preferably greater than 20-fold, preferably greater than 30-fold, preferably greater than 40-fold and preferably greater than 50-fold inhibition of the cellular shedding of all substrates tested compared to the reference compounds.

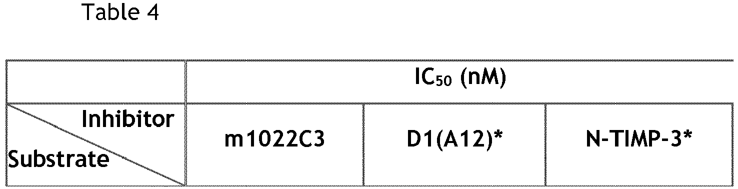

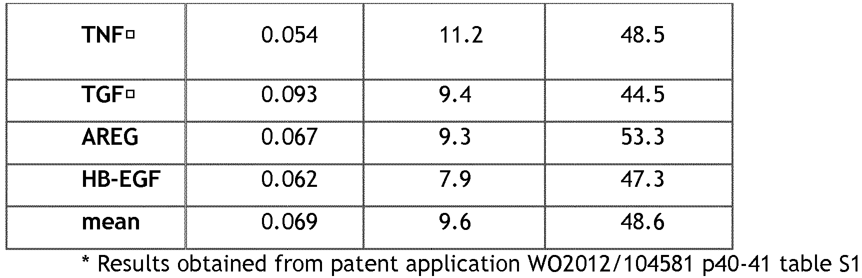

### Example 4: mAb 1022C3 binding to ADAM17

The binding profile of mAb 1022C3 to human, murine and chimeric ADAM17 was determined by western blot and surface Plasmon resonance. A number of ADAM17 sub domains and human/murine chimeric proteins were expressed as human Fc fusion proteins from HEK293 cells. Protein A purified proteins were tested for binding following SDS-PAGE separation followed by western blot analysis with m1022C3 and by surface plasmon resonance. The proteins produced and tested for binding are detailed in table 4. Amino acid positions are cited with reference to human ADAM17: accession number P78536 and murine ADAM17: accession number AAI38421. Expressed ADAM17 domains of human origin are written in uppercase letters, domains or murine origin are in lower case letters. Domain names are abbreviated as follows: P, pro-domain; C, catalytic domain; D, disintegrin domain; MPD, membrane proximal domain. Fragmented domains are indicated by their position in the protein structure amino-terminally (Nter) or carboxy-terminally (Cter).

### Western blot binding assay:

Equal amounts of purified proteins were resolved by 4-15% SDS-polyacrylamide gel under non-reducing conditions and transferred to nitrocellulose membrane. Blocking was performed by incubating the membrane with 1% non-fat milk in Tris-buffered saline (TBS) containing 0.05% Tween 20 (TBS-T). The membrane was then incubated with 1 µg/ml m1022C3 antibody in TBS-T for 1h at room temperature under continuous agitation and then with horseradish peroxidase-conjugated anti-mouse IgG at a dilution of 1:3000 in TBS-T for 1h at room temperature under continuous agitation. Immunoreactive proteins were visualized by enhanced chemiluminescent detection system kit according to the manufacturer's instructions.

### BIAcore binding assay:

The experiment was performed on a Biacore X100 device. The mAb 1022C3 is used as the ligand and the ADAM17 fragments and chimeric constructs are used as the analyte. The experiment is run at 10µl/min at 25°C on a rabbit anti-mouse polyclonal antibody (Mouse antibody capture kit, BR-1008-38, GE Healthcare) covalently linked to the matrix of both flowcells of a CM5 sensorchip (BR-1000-12) using the amine coupling kit (BR-1000-50, GE Healthcare), using the HBS-EP+ buffer (BR-1008-26, GE Healthcare) as the running buffer. This buffer is also used for the dilutions of the ligand and the analytes.

A solution of the mAb 1022C3 at the concentration of 15µg/ml is injected on the second flowcell (working surface) during 1 minute. At each cycle, one of the ADAM17 chimeric constructions (with a human Fc domain at the c-terminal positions) is injected at the concentration of 250nM during 3 minutes on both flow cells: the reference without any m1022C3 (FC1) and the working cell with around 700 RU of m1022C3 (FC2). The registered signal corresponds to the difference between FC2 and FC1 responses.

The positive response is between 90 and 140 RU. The negative responses are all bellow 10 RU. At the end of each cycle the m1022C3 is removed by a injection of a 10mM Glycine,HCl pH 1.7 buffer (from the Mouse antibody captured kit) during 3 minutes.

**Table 5**

| Amino acid position | Domain | WB | BIAcore |
|---|---|---|---|
| H1-671 | P-C-D-MPD | + | + |
| M1-671 | p-c-d-mpd | - | - |
| H1-474 | P-C | - | - |
| H223-563 | C-D | - | - |
| H475-563 | D | - | - |
| H223-602 | C-D MPD (Nter) | - | - |
| H475-602 | D-MPD (Nter) | - | - |
| H457-671 | D-MPD | + | + |
| H603-671 | MPD (Cter) | - | - |
| M1-602H603-671 | p-c-d-mpd (Nter)-MPD (Cter) | - | - |
| M1-563H564-671 | p-c-d-MPD | + | + |
| M1-474H475-671 | p-c-D-MPD | + | + |

### Example 5: Definition of the dissociation constant of the binding of the extracellular domain of ADAM-17 on monoclonal antibody 1022C3 with Surface Plasmon Resonance experiments.

The antibody (ligand) was bound to the second flowcell of a Biacore CM5 sensor chip (GE Healthcare) activated on both flowcells with a Rabbit anti-Mouse (RAM) IgG (H+L) covalently linked to the carboxymethyldextran matrix. Soluble ADAM17 (analyte) at concentrations ranging from 400 to 12.5 nM obtained by a two-fold dilution scheme (assuming a molecular weight of 52 kDa) was injected onto the surface at a flow rate of 30µl/min in a 120 s pulse (association) plus an extra 180 s delay for the dissociation phase measurement. The RAM surface was regenerated using NaOH 30mM, NaCl 150mM and 10mM Glycine,HCl pH 1.5 buffer solutions. Curves obtained at each concentration were double referenced by first subtracting the signal from the reference FC1 surface (RAM without any mouse anti-TACE mAb) followed by subtraction of the signal obtained from a running buffer injection (Biacore HBS-EP buffer).

Data were processed using BIAevaluation 3.1 software using the 1:1 Langmuir model. The suitability of the fit was measured by the values of the Refractive Index (RI) which have to tend to zero and the κ² value.

The results are summarized in the following table 6

**Table 6**

| Antibodies | Ab capture range (RU) | Rmax range (RU) | RI range (RU) | □² | kₒₙ (1/M.s) | k_{off} (1/s) | K_{D} (nM) |
|---|---|---|---|---|---|---|---|
| 1022C3 | 279.7 269.6 | 120 98.1 | 4.59 -4.57 | 2.27 | 2.84 x 10⁵ ± 2.31 x 10³ | 3.60 x 10⁻⁴ ± 12.3 x 10⁻⁶ | 1.27 ± 0.05 |

### Example 6: Binding evaluation and FRET inhibition of the deglycosylated CDRH1

The mAb 1022C3 possesses an N-glycosylation site, that is post translationally modified in the secreted protein, located in CDRH1. To determine the influence of the glycosylation site m1022C3 was enzymatically deglycosylated a process that converts the Asparagine residue to Aspartic acid.

The mAb m1022C3 was deglycosylated using two glycosidases in a sequential manner. 1 µL of neuraminidase (New England Biolabs, P0720S, 50 000 U/mL) was added to 20 µg of a 1 mg/mL mAb solution and the mixture was incubated under gentle agitation at 37°C overnight. 1 µL of Peptide-N-Glycosidase F (New England Biolabs, P0704S, 500 000 U/mL) was then added following by another incubation step overnight at 37°C.

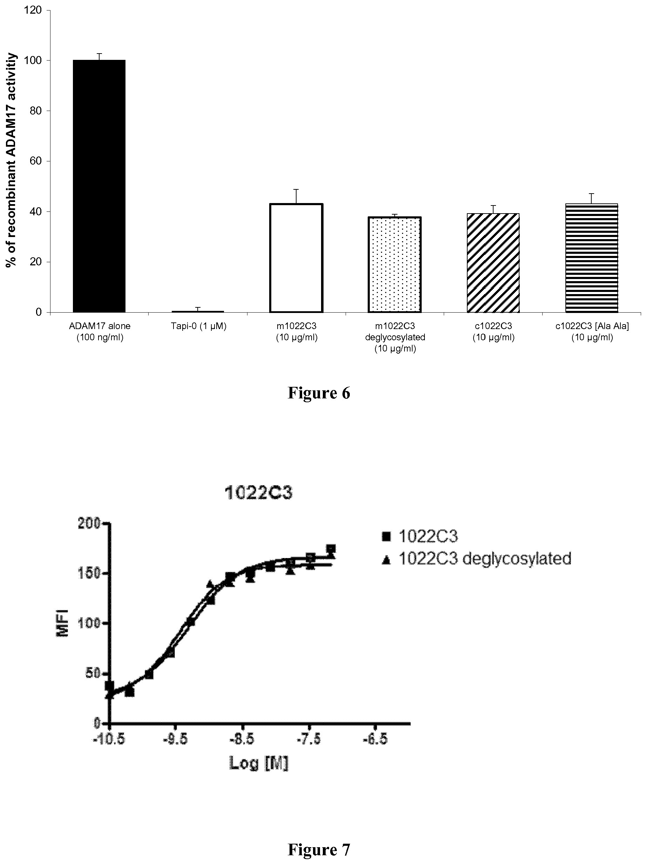

Enzymatic deglycosylation of m1022C3 did not reduce the inhibitory activity of m1022C3 *in vitro* evaluated in a FRET peptide cleavage assay (Figure 6) retaining parental mAb levels of inhibition.

The enzymatically deglycosylated 1022C3 was evaluated for binding to the tumour cell line NCI-H1299 and was shown to have retained the binding capacity of the parental antibody (Figure 7).

### Example 7: Amphiregulin shedding assay

ADAM17 is involved in the shedding of a series of EGFR ligands including amphiregulin (AREG). In order to determine whether the m1022C3 is able to inhibit the cleavage of AREG induced by ADAM17, this ligand has been dosed in the supernatant of MCF-7 oestrogen-dependent breast cancer cells. Briefly, 16 000 MCF-7 cells have been plated in each well of a 96 well plate in 200 µL of complete culture medium (RPMI 1640 w/o phenol red + 10%SVF + 1% L-Glutamine). Forty-eight hours post-plating, medium was removed, cells were washed in PBS (Phosphate Buffer Saline) and compounds to be tested were added in 0.5% SFV medium. In these experiments TAPI1 was introduced as a positive control of inhibition (reference compound) and either mlgG1 or hlgG1 isotype controls were included as negative control depending on the format of the tested antibody. PMA was also used as a positive control of AREG shedding. Cells were then incubated for 48 additional hours before supernatant collection and dosage of AREG by the ELISA DUO SET quantification assay (R&D Systems). At the same time, cell viability was evaluated using a Cell Titer Glo luminescent method in order to be sure that cell death was not be responsible of the AREG release process.

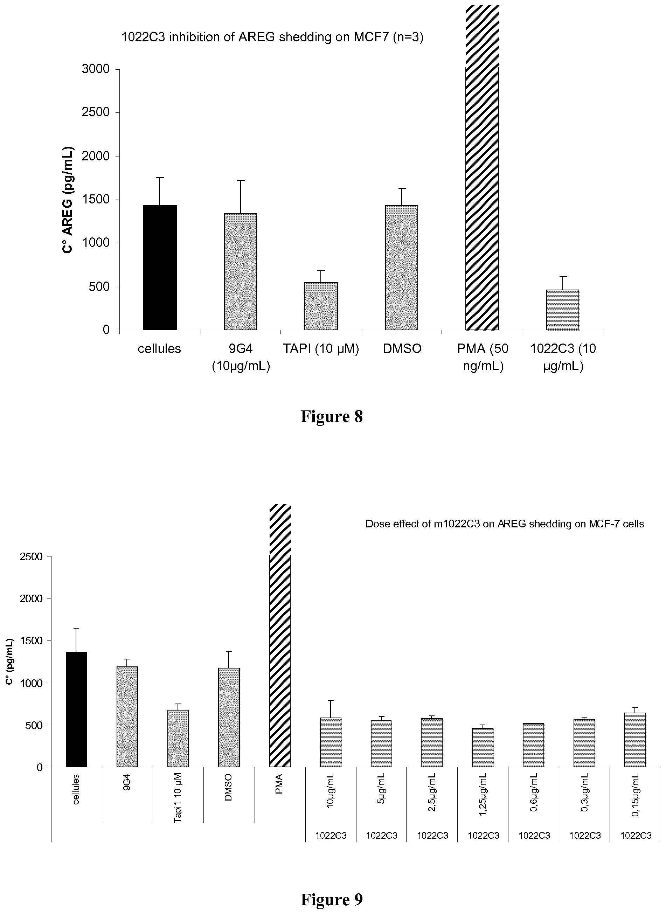

Figure 8 is the mean of 3 independent experiments. It showed that, as expected, the reference compound TAPI1 induced a significant inhibition of AREG shedding (62%) while PMA introduced as a positive control of shedding is able, in this setting to induce a dramatic shedding of AREG. Neither the isotype control, or DMSO, used as a vehicle for TAPI1 solubilisation interfered with the AREG shedding. The control mAb 9G4, introduced as a murine isotype control was without effect on AREG Shedding. m1022C3 inhibited AREG shedding as potently as the reference compound did. A dose range of m1022C3 was then tested (Figure 9). It demonstrated that m1022C3 was active at doses as low as 0.15 µg/ml.

### Example 8: In vivo evaluation of the mAb 1022C3

For all in vivo evaluations, six to eight weeks old athymic mice were used. They were housed in sterilized filter-topped cages, maintained in sterile conditions and manipulated according to French and European guidelines.

ADAM17 expression levels were determined by staining, 1x10⁵ cells/100 µl in FACS buffer (PBS containing 1% BSA and 0.01% sodium azide) incubated for 20 min. at 4°C with increasing concentrations of the MAB9301 (Clone 111633, R&D systems) in order to determine a saturating concentration. Cells were then washed three times in FACS buffer. Cells were resuspended and incubated for 20 min. at 4°C with a goat anti-mouse lgG-Alexa 488 antibody (Invitrogen Corporation, Scotland, # A11017). Cells were then washed three times in FACS buffer. Labelled cells were then resuspended in 100 µl of FACS buffer prior to analysis with a Facscalibur cytometer (Becton Dickinson, Le Pont-de-Claix, France). Propidium iodide was added to analyse only viable cells. In parallel, QIFIKIT beads were used for the determination of antibody-binding and antigen density per cell by flow cytometry and monoclonal antibody binding. QIFIKIT contains a series of beads, 10 µm in diameter and coated with different, but well-defined quantities of mouse mAb molecules. The beads mimic cells with different antigen densities which have been labelled with a primary mouse mAb. The quantified antigen is expressed in Antibody-Binding Capacity (ABC) units.

### 8.1 A431 xenograft model: established tumours

A431, an epidermoid carcinoma cell line, expressing ADAM17 (ABC = 17 000), was selected for *in vivo* evaluations. Mice were injected subcutaneously at D0 with 10x10⁶ cells. When tumours reached approximately 200 mm³ (20 days post tumour cell injection), animals were divided into two groups of 6 mice with comparable tumour size and treated intraperitoneally with a loading dose of 20 mg/kg and then weekly with maintenance doses of 10 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

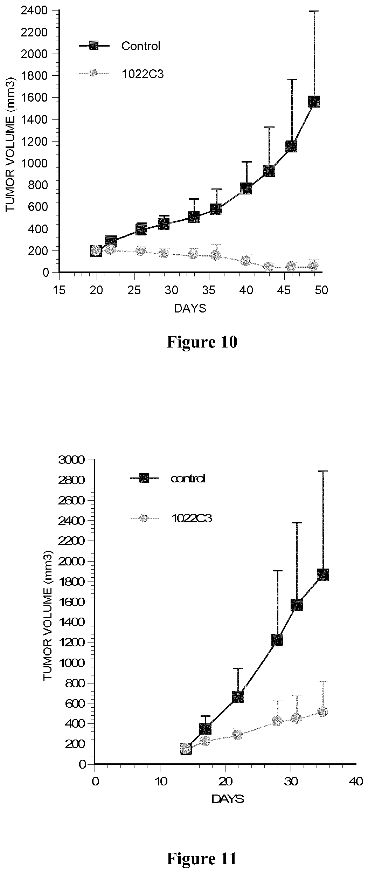

The results obtained were summarized in Figure 10. They showed a dramatic tumour inhibition (97% at D49) mediated by the m1022C3 mAb with tumour regressions observed in all treated mice and complete regressions achieved in 2 out of 6 mice at D49.

### 8.2 A431 xenograft model established with tumour fragments

In order to determine the robustness of the observed antitumour activity of the mAb m1022C3 , tumours were first generated by cell implantation as described above. Then when tumour volume reached approximately 200-300 mm³, tumours were removed aseptically, minced in 1 mm³ fragments, carefully avoiding necrotic areas, and these fragments were then used for tumour propagation in a new series of athymic mice. This propagation cycle was performed 3 times in order to stabilize both tumour growth and characteristics and then 2 groups of 6 mice bearing tumours reaching approximately 145 mm³ (14 days post tumour cell injection), were generated. One group was treated intraperitoneally with a loading dose of 20 mg/kg and then weekly with maintenance doses of 10 mg/kg of m1022C3 monoclonal antibody. The second group received only the vehicle. The mice were followed for the observation of xenograft growth rate. Tumour volume was calculated by the formula: π/6 X length X width X height.

The results obtained were summarized in Figure 11. Tumours established from fragments grow faster than those resulting from cell engraftment. This observation was in agreement with a more aggressive phenotype of these tumours. In this more aggressive setting the m1022C3 is still significantly active with a tumour inhibition reaching 73% at day 35.

### 8.3 Comparison of the murine 1022C3 (m1022C3) with its lgG1 chimeric form (c1022C3) on the A431 xenograft model.

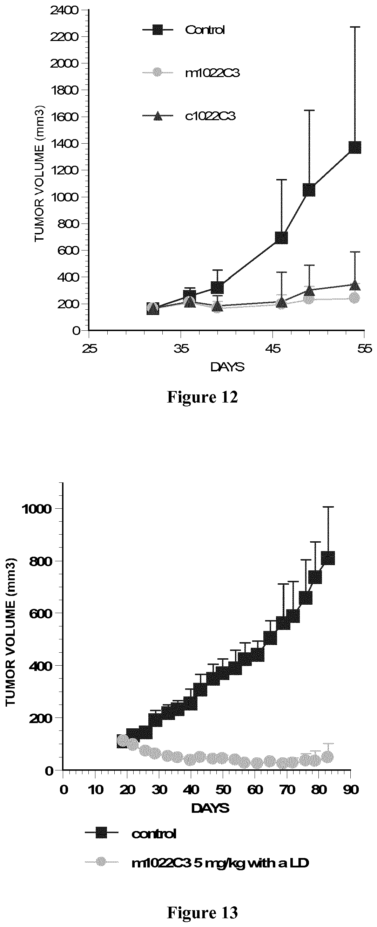

In order to compare the m1022C3 with its chimeric form, the A431 xenograft model was set up by cell engraftments on athymic mice as described above. Results presented in figure 12 demonstrated that the two compounds are comparable with tumour inhibitions reaching respectively 82% and 75% for m1022C3 and c1022C3.

### 8.4 CaOV3 xenograft model: established tumours

CaOV-3, an ovarian carcinoma cell line, expressing ADAM17 (ABC = 20 000), was selected for *in vivo* evaluations. Mice were injected subcutaneously at D0 with 7x10⁶ cells. When tumours reached approximately 120 mm³ (18 days post tumour cell injection), animals were divided into two groups of 5 mice with comparable tumour size and treated intraperitoneally with a loading dose of 10 mg/kg and then weekly with maintenance doses of 5 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

The results obtained were summarized in Figure 13. They showed a dramatic tumour inhibition mediated by the m1022C3 mAb with tumour regressions observed in all treated mice and complete regressions achieved in 1 out of 5 mice since D69. The tumour inhibition reached 94% at D84.

### 8.5 OVISE xenograft model: established tumours

OVISE, an ovarian carcinoma cell line, expressing ADAM17 (ABC = 49000), was selected for *in vivo* evaluations. Mice were injected subcutaneously at D0 with 7x10⁶ cells. When tumours reached approximately 100 mm³ (28 days post tumour cell injection), animals were divided into two groups of 6 mice with comparable tumour size and treated intraperitoneally with a loading dose of 10 mg/kg and then weekly with maintenance doses of 5 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

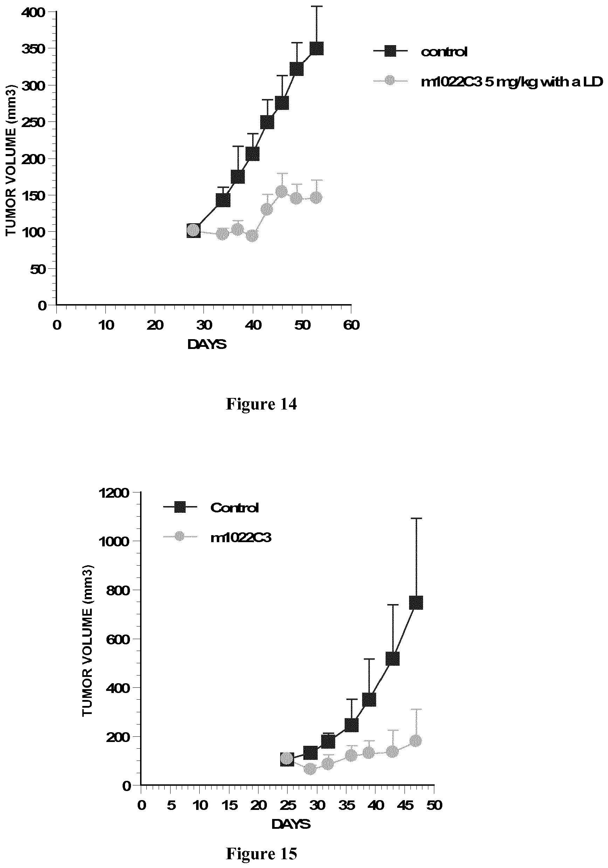

The results obtained were summarized in Figure 14. They showed a dramatic tumour inhibition mediated by the mAb m1022C3 with tumour regressions observed in all treated mice with a tumour inhibition reaching 58% at D53.

### 8.6_BxPC3 xenograft tumours

BxPC3, a pancreatic carcinoma cell line, expressing ADAM17 (ABC = 12 600), was selected for *in vivo* evaluations. Mice were injected subcutaneously at D0 with 7x10⁶ cells. When tumours reached approximately 100 mm³ (25 days post tumour cell injection), animals were divided into two groups of 6 mice with comparable tumour size and treated intraperitoneally with a loading dose of 20 mg/kg and then weekly with maintenance doses of 10 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

The results obtained were summarized in Figure 15. They showed a dramatic tumour inhibition, reaching 76% at D47 mediated by the mAb m1022C3.

### 8.7 BICR-22 xenograft model: established tumours

BICR-22, a tongue squamous carcinoma cell line, expressing ADAM17 (ABC = 16 000), was selected for *in vivo* evaluations. Mice were injected subcutaneously at D0 with 7x10⁶ cells. When tumours reached approximately 100 mm³ (11 days post tumour cell injection), animals were divided into two groups of 6 mice with comparable tumour size and treated intraperitoneally with a loading dose of 10 mg/kg and then weekly with maintenance doses of 5 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

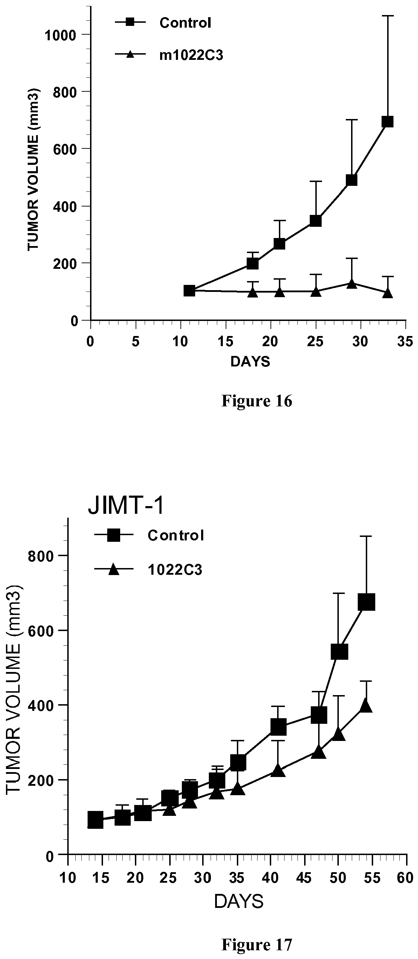

The results obtained were summarized in Figure 16. They showed a dramatic tumour inhibition (86% at D33) mediated by the m1022C3 mAb.

### 8.8 JIMT-1 xenograft model: established tumours

JIMT-1, a breast carcinoma cell line known to be resistant to a HER2 targetted therapy, expressing ADAM17 (ABC = 30 000), was selected for *in vivo* evaluations. Mice were injected subcutaneously at D0 with 7x10⁶ cells. When tumours reached approximately 100 mm³ (14 days post tumour cell injection), animals were divided into two groups of 6 mice with comparable tumour size and treated intraperitoneally with a loading dose of 20 mg/kg and then weekly with maintenance doses of 10 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

The results obtained were summarized in Figure 17. They showed a tumour inhibition (41% at D33) mediated by the m1022C3 mAb.

### 8.9 NCI-H292 xenograft model: established tumours

NCI-H292, a mucoepidermoid pulmonary carcinoma cell line, expressing ADAM17 (ABC = 12 000), was selected for *in vivo* evaluations. Mice were injected subcutaneously at D0 with 7x10⁶ cells. When tumours reached approximately 150 mm³ (9 days post tumour cell injection), animals were divided into two groups of 6 mice with comparable tumour size and treated intraperitoneally with a loading dose of 20 mg/kg and then weekly with maintenance doses of 10 mg/kg of m1022C3 monoclonal antibody. A control group received only the vehicle as previous experiments performed in this model demonstrated that no difference in tumour growth was observed between mice treated with vehicle and mice injected with an isotype control. The mice were followed for the observation of xenograft growth rate. Tumour volume was calculated by the formula: π/6 X length X width X height.

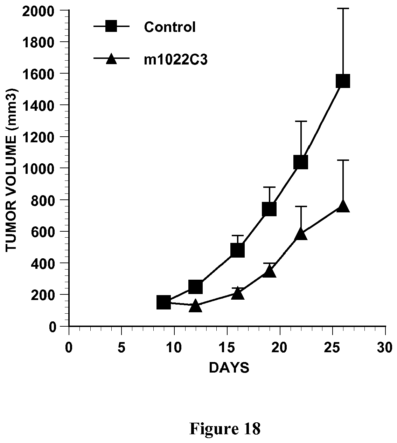

The results obtained were summarized in Figure 18. They showed a tumour inhibition (50% at D26) mediated by the m1022C3 mAb.

## Claims

1. An anti-ADAM17 monoclonal antibody, or an antigen-binding fragment thereof, comprising:
i) a heavy chain comprising CDR-H1, CDR-H2 and CDR-H3 of amino acid sequences SEQ ID Nos. 1, 2 and 3, respectively; and
ii) a light chain comprising CDR-L1, CDR-L2 and CDR-L3 of amino acid sequences SEQ ID Nos. 4, 5 and 6, respectively.

2. The antibody of claim 1,wherein the amino acid sequence of CDR-H1 is SEQ ID Nos. 7 or 8.

3. The antibody of claim 1, said antibody comprising a heavy chain variable domain comprising the amino acid sequence SEQ ID No. 9, 11 or 12.

4. The antibody of claim 1, said antibody comprising a light chain variable domain comprising the amino acid sequence SEQ ID No. 10.

5. The antibody of claim 1, said antibody comprising:
• a heavy chain variable domain comprising the amino acid sequence SEQ ID No. 9, 11 or 12; and
• a light chain variable domain comprising the amino acid sequence SEQ ID No. 10.

6. The monoclonal antibody 1022C3, or an antigen-binding fragment thereof, produced from the hybridoma I-4686 deposited at the CNCM, Institut Pasteur, France, on the 18 October 2012.

7. The antibody of any one of claims 1 to 6, wherein said antibody is capable of binding to a single epitope of ADAM17 within the membrane proximal domain (MPD) of sequence SEQ ID No. 32.

8. The antibody of claim 7, wherein said antibody inhibits the shedding of at least one substrate of ADAM17 with an IC₅₀ of 500 pM or less, preferentially 200 pM or less and more preferentially 100 pM or less.

9. The antibody of claim 7, wherein said at least one substrate of ADAM17 is selected from TNF-α, TGF-α, AREG and HB-EGF.

10. The antibody of claim 9, wherein said antibody binds to an ADAM17 epitope with a Kd of about 10 nM or less, preferentially of about 5 nM or less, as determined by surface plasmon resonance (SPR).

11. The murine hybridoma I-4686 deposited at the CNCM, Institut Pasteur, France, on the 18 October 2012.

12. An isolated nucleic acid, coding for the antibody, or antigen-binding fragment thereof, of any one of claims 1 to 10.

13. The isolated nucleic acid of claim 12, said nucleic acid comprising at least one sequence selected from the group consisting of SEQ ID No. 13-28 and 36 to 38.

14. A vector comprising a nucleic acid of any one of claims 12 or 13.

15. A host cell comprising the vector of claim 14.

16. A non-human transgenic animal comprising at least one cell transformed by a vector of claim 14.

17. A method for producing the antibody, or antigen-binding fragment thereof, of any one of claims 1 to 10, said method comprising the steps of:
a) growing the cell of claim 15 in a medium and appropriate culture conditions; and
b) recovering said antibody, or antigen-binding fragment thereof, thus produced from the culture medium or said cultured cells.

18. A composition comprising the antibody according to one of claims 1 to 10.

19. The composition of claim 18, further comprising a cytotoxic agent, as a combination product or a conjugation product.

20. The composition of any one of claims 18 or 19, further comprising a pharmaceutically acceptable vehicle.

21. The antibody of any one of claims 1 to 10 or the composition of any one of claims 18 to 20 for use in a method for inhibiting the growth and/or the proliferation of tumour cells.

22. The antibody of any one of claims 1 to 10 or of a composition according to one of claims 18 to 20 for use in the treatment of cancer.

23. The antibody or the composition for the use of claim 22, wherein said cancer is a cancer expressing ADAM17.

24. The antibody or the composition for the use of any one of claims 22 or 23, wherein said cancer is a cancer chosen from ovarian cancer, breast cancer, kidney cancer, liver cancer, pancreatic cancer, lung cancer, head and neck cancer and epidermoid carcinoma.

## Patentansprüche

1. Monoklonaler Anti-ADAM17-Antikörper oder ein Antigen-bindendes Fragment davon, umfassend:
i) eine schwere Kette, umfassend jeweils CDR-H1, CDR-H2 und CDR-H3 der Aminosäuresequenzen SEQ ID NR. 1, 2 und 3; und;
ii) eine leichte Kette, umfassend jeweils CDR-L1, CDR-L2 und CDR-L3 der Aminosäuresequenzen SEQ ID NR. 4, 5 und 6.

2. Antikörper gemäß Anspruch 1, wobei die Aminosäuresequenz von CDR-H1 die SEQ ID NR. 7 oder 8 ist.

3. Antikörper gemäß Anspruch 1, umfassend eine Schwere-Ketten-variable Domäne, umfassend die Aminosäuresequenz SEQ ID NR. 9, 11 oder 12.

4. Antikörper gemäß Anspruch 1, wobei der genannte Antikörper eine Leichte-Ketten-variable-Domäne umfasst, umfassend die Aminosäure-Sequenz SEQ ID NR. 10.

5. Antikörper gemäß Anspruch 1, wobei der Antikörper umfasst:
• eine Schwere-Ketten-variable-Domäne, umfassend die Aminosäuresequenz SEQ ID NR. 9, 11 oder 12; und
• eine Leichte-Ketten-variable-Domäne, umfassend die Aminosäuresequenz SEQ ID NR. 10.

6. Monoklonaler Antikörper 1022C3 oder ein Antigen-bindendes Fragment davon, das von dem Hybridom I-4686 produziert wurde, das am 18. Oktober 2012 beim CNCM, Institut Pasteur, Frankreich, angemeldet wurde.

7. Antikörper gemäß irgendeinem der Ansprüche 1 bis 6, wobei der genannte Antikörper zum Binden eine einzelnen Epitops von ADAM17 innerhalb der Membranproximalen-Domäne (MPD) der Sequenz SEQ ID NR. 32 fähig ist.

8. Antikörper gemäß Anspruch 7, wobei der genannte Antikörper die Fachbildung wenigstens eines Substrats des ADAM17 mit einem IC₅₀ von 500 pM oder weniger, bevorzugt 200 pM oder weniger und noch weiter bevorzugt 100 pM oder weniger inhibiert.

9. Antikörper gemäß Anspruch 7, wobei das genannte wenigstens eine Substrat des ADAM17 ausgewählt ist aus TNF-α, TGF-α, AREG und HB-EGF.

10. Antikörper gemäß Anspruch 9, wobei sich der genannte Antikörper an ein ADAM17-Epitop mit einem Kd von ungefähr 10 nM oder weniger, bevorzugt von ungefähr 5 nM oder weniger gemäß Bestimmung per Oberflächen-Plasmonresonanz (SPR) bindet.

11. Murines Hybridom I-4686, das am 18. Oktober 2012 beim CNCM, Institut Pasteur, France, angemeldet wurde.

12. Isolierte Nukleinsäure, die für den Antikörper kodiert, oder ein Antigen-bindendes Fragment davon, gemäß irgendeinem der Ansprüche 1 bis 10.

13. Isolierte Nukleinsäure gemäß Anspruch 12, wobei die genannte Nukleinsäure wenigstens eine Sequenz umfasst, die ausgewählt ist aus der Gruppe bestehend aus der SEQ ID NR. 13-28 und 36 bis 38.

14. Vektor, umfassend eine Nukleinsäure gemäß irgendeinem der Ansprüche 12 oder 13.

15. Wirtszelle, umfassend den Vektor gemäß Anspruch 14.

16. Nicht-menschliches, transgenes Tier, umfassend wenigstens eine Zelle, die durch einen Vektor gemäß Anspruch 14 transformiert ist.

17. Verfahren zur Produktion des Antikörpers oder Antigen-bindenden Fragments davon gemäß irgendeinem der Ansprüche 1 bis 10, wobei das genannte Verfahren die Schritte umfasst:
a) Züchten einer Zelle gemäß Anspruch 15 in einem Medium und unter geeigneten Kulturbedingungen; und
b) Zurückgewinnen des genannten Antikörpers oder Antigen-bindenden Fragments davon, der / das somit aus dem Kulturmedium oder den genannten gezüchteten Zellen produziert wurde.

18. Zusammensetzung, umfassend den Antikörper gemäß einem der Ansprüche 1 bis 10.

19. Zusammensetzung gemäß Anspruch 18, weiterhin umfassend einen zytotoxischen Wirkstoff als ein Kombinationsprodukt oder ein Konjugationsprodukt.

20. Zusammensetzung gemäß irgendeinem der Ansprüche 18 oder 19, weiterhin umfassend eine pharmazeutisch akzeptablen Träger.

21. Antikörper gemäß irgendeinem der Ansprüche 1 bis 10 oder Zusammensetzung gemäß irgendeinem der Ansprüche 18 bis 20 zur Verwendung in einem Verfahren zum Inhibieren des Wachstums und / oder der Proliferation von Tumorzellen.

22. Antikörper gemäß irgendeinem der Ansprüche 1 bis 10 oder Zusammensetzung gemäß einem der Ansprüche 18 bis 20 zur Verwendung bei der Behandlung von Krebserkrankungen.

23. Antikörper oder Zusammensetzung zur Verwendung gemäß Anspruch 22, wobei die genannte Krebserkrankung eine ADAM17 exprimierende Krebserkrankung ist.

24. Antikörper oder Zusammensetzung zur Verwendung gemäß irgendeinem der Ansprüche 22 oder 23, wobei die genannte Krebserkrankung eine Krebserkrankung ist, die ausgewählt ist aus Eierstockkrebs, Brustkrebs, Nierenkrebs, Leberkrebs, Bauchspeicheldrüsenkrebs, Lungenkrebs, Kopf- und Halskrebs und Carcinoma epidermoidale.

## Revendications

1. Anticorps monoclonal anti-ADAM17, ou son fragment de liaison à l'antigène, comprenant :
i) une chaîne lourde qui comprend CDR-H1, CDR-H2 et CDR-H3 respectivement de séquences d'acides aminés SEQ ID Nos. 1, 2 et 3 ; et
ii) une chaîne légère qui comprend CDR-L1, CDR-L2 et CDR-L3 respectivement de séquences d'acides aminés SEQ ID Nos. 4, 5 et 6.

2. Anticorps selon la revendication 1, dans lequel la séquence d'acides aminés de CDR-H1 est SEQ ID No. 7 ou 8.

3. Anticorps selon la revendication 1, ledit anticorps comprenant un domaine variable de chaîne lourde qui comprend la séquence d'acides aminés SEQ ID No. 9, 11 ou 12.

4. Anticorps selon la revendication 1, dans lequel ledit anticorps comprend un domaine variable de chaîne légère qui comprend la séquence d'acides aminés SEQ ID No. 10.

5. Anticorps selon la revendication 1, ledit anticorps comprenant :
• un domaine variable de chaîne lourde qui comprend la séquence d'acides aminés SEQ ID No. 9, 11 ou 12 ; et
• un domaine variable de chaîne légère qui comprend la séquence d'acides aminés SEQ ID No. 10.

6. Anticorps monoclonal 1022C3, ou son fragment de liaison à l'antigène, produit à partir de l'hybridome I-4686 déposé au CNCM, institut Pasteur, France, le 18 octobre 2012.

7. Anticorps selon l'une quelconque des revendications 1 à 6, dans lequel ledit anticorps dispose de la capacité de se lier à un unique épitope d'ADAM17 à l'intérieur du domaine proximal de membrane (MPD) de la séquence SEQ ID No. 32.

8. Anticorps selon la revendication 7, dans lequel ledit anticorps inhibe l'excrétion d'au moins un substrat d'ADAM17 avec un IC₅₀ de 500 pM ou moins, préférablement de 200 pM ou moins et plus préférablement préférable, de 100 pM ou moins.

9. Anticorps selon la revendication 7, dans lequel ledit au moins un substrat d'ADAM17 est sélectionné parmi TNF-α, TGF-α, AREG et HB-EGF.

10. Anticorps selon la revendication 9, dans lequel ledit anticorps se lie à un épitope d'ADAM17 avec un Kd d'environ 10 nM ou moins, préférablement d'environ 5 nM ou moins, comme déterminé au moyen de la résonance plasmonique de surface (SPR).

11. Hybridome murin I-4686 déposé au CNCM, institut Pasteur, France, le 18 octobre 2012.

12. Acide nucléique isolé, codant l'anticorps, ou son fragment de liaison à l'antigène, selon l'une quelconque des revendications 1 à 10.

13. Acide nucléique isolé selon la revendication 12, ledit acide nucléique comprenant au moins une séquence qui est sélectionnée parmi le groupe qui est constitué par SEQ ID Nos. 13-28 et 36 à 38.

14. Vecteur comprenant un acide nucléique selon l'une quelconque des revendications 12 ou 13.

15. Cellule hôte comprenant le vecteur de la revendication 14.

16. Animal transgénique non humain comprenant au moins une cellule transformée au moyen d'un vecteur selon la revendication 14.

17. Procédé de production de l'anticorps, ou son fragment de liaison à l'antigène, selon l'une quelconque des revendications 1 à 10, comprenant les étapes constituées par :
a) la croissance de la cellule selon la revendication 15 dans un milieu et sous des conditions de culture appropriées ; et
b) la récupération dudit anticorps, ou de son fragment de liaison à l'antigène, ainsi produit à partir du milieu de culture ou desdites cellules mises en culture.

18. Composition comprenant l'anticorps selon l'une des revendications 1 à 10.

19. Composition selon la revendication 18, comprenant en outre un agent cytotoxique, en tant que produit de combinaison ou que produit de conjugaison.

20. Composition selon l'une quelconque des revendications 18 ou 19, comprenant en outre un véhicule pharmaceutiquement acceptable.

21. Anticorps selon l'une quelconque des revendications 1 à 10 ou composition selon l'une quelconque des revendications 18 à 20 pour une utilisation pour inhiber la croissance et/ou la prolifération de cellules tumorales.

22. Anticorps selon l'une quelconque des revendications 1 à 10 ou composition selon l'une des revendications 18 à 20 pour une utilisation pour le traitement du cancer.

23. Anticorps ou composition pour l'utilisation selon la revendication 22, dans laquelle ledit cancer est un cancer qui exprime ADAM17.

24. Anticorps ou composition pour l'utilisation selon l'une quelconque des revendications 22 ou 23, dans laquelle ledit cancer est un cancer choisi parmi le cancer des ovaires, le cancer du sein, le cancer du rein, le cancer du foie, le cancer pancréatique, le cancer du poumon, le cancer de la tête et du cou et le carcinome épidermoïde.


### SEQUENCE LISTING

<110> PIERRE FABRE MEDICAMENT
<120> NOVEL ANTI ADAM17 ANTIBODY AND ITS USE FOR THE TREATMENT OF CANCER
<130> 366742D33276
<150> EP13306860.1
   <151> 2013-12-24
<160> 38
<170> PatentIn version 3.5
<210> 1
   <211> 10
   <212> PRT
   <213> artificial
<220>
   <223> 1022C3 antibody CDR-H1 (X1)
<220>
   <221> VARIANT
   <222> (5)..(5)
   <223> X is N, D, Q, S, E, R, K, H, W, Y or T
<400> 1
   Gly Phe Ser Leu Xaa Thr Ser Gly Met Gly
   1               5                   10
<210> 2
   <211> 7
   <212> PRT
   <213> artificial
<220>
   <223> 1022C3 antibody CDR-H2
<400> 2
   Ile Tyr Trp Asp Asp Asp Lys
   1 5
<210> 3
   <211> 15
   <212> PRT
   <213> artificial
<220>
   <223> 1022C3 antibody CDR-H3
<400> 3
   Ala Arg Arg Ser His Phe Gly Asn Tyr Tyr Tyr Ala Met Asp Tyr
   1 5 10 15
<210> 4
   <211> 6
   <212> PRT
   <213> artificial
<220>
   <223> 1022C3 antibody CDR-L1
<400> 4
   Gln Asp Ile Ser Ser Tyr
   1 5
<210> 5
   <211> 3
   <212> PRT
   <213> artificial
<220>
   <223> 1022C3 antibody CDR-L2
<400> 5
   Tyr Thr Ser
   1
<210> 6
   <211> 9
   <212> PRT
   <213> artificial
<220>
   <223> 1022C3 antibody CDR-L3
<400> 6
   Gln Gln Gly Lys Thr Leu Pro Leu Thr
   1 5
<210> 7
   <211> 10
   <212> PRT
   <213> artificial
<220>
   <223> 1022C3 antibody CDR-H1 (N)
<400> 7
   Gly Phe Ser Leu Asn Thr Ser Gly Met Gly
   1               5                   10
<210> 8
   <211> 10
   <212> PRT
   <213> artificial
<220>
   <223> 1022C3 antibody CDR-H1 (D)
<400> 8
   Gly Phe Ser Leu Asp Thr Ser Gly Met Gly
   1               5                   10
<210> 9
   <211> 123
   <212> PRT
   <213> artificial
<220>
   <223> 1022C3 antibody heavy chain variable domain (X1)
<220>
   <221> VARIANT
   <222> (30)..(30)
   <223> X is N, D, Q, S, E, R, K, H, W, Y or T
<400> 9
   Gln Val Thr Leu Lys Glu Ser Gly Pro Gly Ile Leu Gln Pro Ser Gln
   1               5                   10                  15
   Thr Leu Ser Leu Thr Cys Ser Phe Ser Gly Phe Ser Leu Xaa Thr Ser
               20                  25                  30
   Gly Met Gly Val Ser Trp Ile Arg Gln Pro Ser Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Ser Asn Gln Val
   65                  70                  75                  80
   Phe Leu Lys Ile Thr Ser Leu Asp Thr Ala Asp Thr Ala Thr Tyr Tyr
                   85                  90                  95
   Cys Ala Arg Arg Ser His Phe Gly Asn Tyr Tyr Tyr Ala Met Asp Tyr
               100                 105                 110
   Trp Gly Gln Gly Thr Ser Val Thr Val Thr Ser
           115                 120
<210> 10
   <211> 107
   <212> PRT
   <213> artificial
<220>
   <223> 1022C3 antibody light chain variable domain
<400> 10
   Asp Val Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Ser Tyr
               20                  25                  30
   Leu Asn Trp Tyr Gln Gln Lys Pro Asp Gly Thr Phe Lys Leu Leu Ile
           35                  40                  45
   Phe Tyr Thr Ser Arg Leu Gln Ser Gly Val Pro Ser Lys Tyr Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Ala Tyr Phe Cys Gln Gln Gly Lys Thr Leu Pro Leu
                   85                  90                  95
   Thr Phe Gly Ala Gly Thr Lys Leu Glu Leu Lys
               100                 105
<210> 11
   <211> 123
   <212> PRT
   <213> artificial
<220>
   <223> 1022C3 antibody heavy chain variable domain (N)
<400> 11
   Gln Val Thr Leu Lys Glu Ser Gly Pro Gly Ile Leu Gln Pro Ser Gln
   1               5                   10                  15
   Thr Leu Ser Leu Thr Cys Ser Phe Ser Gly Phe Ser Leu Asn Thr Ser
               20                  25                  30
   Gly Met Gly Val Ser Trp Ile Arg Gln Pro Ser Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Ser Asn Gln Val
   65                  70                  75                  80
   Phe Leu Lys Ile Thr Ser Leu Asp Thr Ala Asp Thr Ala Thr Tyr Tyr
                   85                  90                  95
   Cys Ala Arg Arg Ser His Phe Gly Asn Tyr Tyr Tyr Ala Met Asp Tyr
   100 105 110
   Trp Gly Gln Gly Thr Ser Val Thr Val Thr Ser
   115 120
<210> 12
   <211> 123
   <212> PRT
   <213> artificial
<220>
   <223> 1022C3 antibody heavy chain variable domain (D)
<400> 12
   Gln Val Thr Leu Lys Glu Ser Gly Pro Gly Ile Leu Gln Pro Ser Gln
   1 5 10 15
   Thr Leu Ser Leu Thr Cys Ser Phe Ser Gly Phe Ser Leu Asp Thr Ser
   20 25 30
   Gly Met Gly Val Ser Trp Ile Arg Gln Pro Ser Gly Lys Gly Leu Glu
   35 40 45
   Trp Leu Ala His Ile Tyr Trp Asp Asp Asp Lys Arg Tyr Asn Pro Ser
   50 55 60
   Leu Lys Ser Arg Leu Thr Ile Ser Lys Asp Thr Ser Ser Asn Gln Val
   65 70 75 80
   Phe Leu Lys Ile Thr Ser Leu Asp Thr Ala Asp Thr Ala Thr Tyr Tyr
   85 90 95
   Cys Ala Arg Arg Ser His Phe Gly Asn Tyr Tyr Tyr Ala Met Asp Tyr
   100 105 110
   Trp Gly Gln Gly Thr Ser Val Thr Val Thr Ser
   115 120
<210> 13
   <211> 30
   <212> DNA
   <213> artificial
<220>
   <223> 1022C3 antibody CDR-H1 (X2)
<220>
   <221> misc_feature
   <222> (13)..(15)
   <223> nnn is AAC, AAT, GAC, GAT, CAG, CAA, TCG, TCT, TCA, TCC, AGT, AGC, GAA, GAG, CGA, CGT, CGC, CGG, AGA, AGG, AAA, AAG, CAT, CAC, TGG, TAT, TAC, ACT, ACC, ACG or ACA
<400> 13
   gggttttcac tgnnnacttc tggtatgggt 30
<210> 14
   <211> 21
   <212> DNA
   <213> artificial
<220>
   <223> 1022C3 antibody CDR-H2
<400> 14
   atttactggg atgacgacaa g 21
<210> 15
   <211> 45
   <212> DNA
   <213> artificial
<220>
   <223> 1022C3 antibody CDR-H3
<400> 15
   gctcgaagat ctcactttgg taactattac tatgctatgg actac 45
<210> 16
   <211> 18
   <212> DNA
   <213> artificial
<220>
   <223> 1022C3 antibody CDR-L1
<400> 16
   caggacatta gtagttat 18
<210> 17
   <211> 9
   <212> DNA
   <213> artificial
<220>
   <223> 1022C3 antibody CDR-L2
<400> 17
   tacacatca 9
<210> 18
   <211> 27
   <212> DNA
   <213> artificial
<220>
   <223> 1022C3 antibody CDR-L3
<400> 18
   caacagggta aaacacttcc gctcacg 27
<210> 19
   <211> 30
   <212> DNA
   <213> artificial
<220>
   <223> 1022C3 antibody CDR-H1 (aac)
<400> 19
   gggttttcac tgaacacttc tggtatgggt 30
<210> 20
   <211> 30
   <212> DNA
   <213> artificial
<220>
   <223> 1022C3 antibody CDR-H1 (aat)
<400> 20
   gggttttcac tgaatacttc tggtatgggt 30
<210> 21
   <211> 30
   <212> DNA
   <213> artificial
<220>
   <223> 1022C3 antibody CDR-H1 (gac)
<400> 21
   gggttttcac tggacacttc tggtatgggt 30
<210> 22
   <211> 30
   <212> DNA
   <213> artificial
<220>
   <223> 1022C3 antibody CDR-H1 (gat)
<400> 22
   gggttttcac tggatacttc tggtatgggt 30
<210> 23
   <211> 369
   <212> DNA
   <213> artificial
<220>
   <223> 1022C3 antibody heavy chain variable domain (X2)
<220>
   <221> misc_feature
   <222> (88)..(90)
   <223> nnn is AAC, AAT, GAC, GAT, CAG, CAA, TCG, TCT, TCA, TCC, AGT, AGC, GAA, GAG, CGA, CGT, CGC, CGG, AGA, AGG, AAA, AAG, CAT, CAC, TGG, TAT, TAC, ACT, ACC, ACG or ACA
<400> 23
   caggttactc tgaaagagtc tggccctggg atattgcagc cctcccagac cctcagtctg      60
   acttgttctt tctctgggtt ttcactgnnn acttctggta tgggtgtgag ctggattcgt     120
   cagccttcag gaaagggtct ggagtggctg gcacacattt actgggatga cgacaagcgc     180
   tataatccat ccctgaagag ccggctcaca atctccaagg atacctccag caaccaggtt     240
   ttcctcaaga tcaccagttt ggacactgca gatactgcca catactactg tgctcgaaga     300
   tctcactttg gtaactatta ctatgctatg gactactggg gtcaaggaac ctcagtcacc     360
   gtcacctca                                                             369
<210> 24
   <211> 369
   <212> DNA
   <213> artificial
<220>
   <223> 1022C3 antibody heavy chain variable domain (aac)
<400> 24
   caggttactc tgaaagagtc tggccctggg atattgcagc cctcccagac cctcagtctg      60
   acttgttctt tctctgggtt ttcactgaac acttctggta tgggtgtgag ctggattcgt     120
   cagccttcag gaaagggtct ggagtggctg gcacacattt actgggatga cgacaagcgc     180
   tataatccat ccctgaagag ccggctcaca atctccaagg atacctccag caaccaggtt     240
   ttcctcaaga tcaccagttt ggacactgca gatactgcca catactactg tgctcgaaga     300
   tctcactttg gtaactatta ctatgctatg gactactggg gtcaaggaac ctcagtcacc     360
   gtcacctca                                                             369
<210> 25
   <211> 369
   <212> DNA
   <213> artificial
<220>
   <223> 1022C3 antibody heavy chain variable domain (gat)
<400> 25
   caggttactc tgaaagagtc tggccctggg atattgcagc cctcccagac cctcagtctg        60
   acttgttctt tctctgggtt ttcactggat acttctggta tgggtgtgag ctggattcgt       120
   cagccttcag gaaagggtct ggagtggctg gcacacattt actgggatga cgacaagcgc       180
   tataatccat ccctgaagag ccggctcaca atctccaagg atacctccag caaccaggtt       240
   ttcctcaaga tcaccagttt ggacactgca gatactgcca catactactg tgctcgaaga       300
   tctcactttg gtaactatta ctatgctatg gactactggg gtcaaggaac ctcagtcacc       360
   gtcacctca                                                               369
<210> 26
   <211> 321
   <212> DNA
   <213> artificial
<220>
   <223> 1022C3 antibody light chain variable domain
<400> 26
   gatgtccaga tgacacagac tacatcctcc ctgtctgcct ctctgggaga cagagtcacc        60
   atcagttgca gggcaagtca ggacattagt agttatttaa attggtatca gcagaaacca       120
   gatggaactt ttaaactcct gatcttctac acatcaagat tacagtcagg agtcccatca       180
   aaatacagtg gcagtgggtc tggaacagat tattctctca ccattagtaa cctggagcaa       240
   gaagatattg ccgcttactt ttgccaacag ggtaaaacac ttccgctcac gttcggggct       300
   gggaccaagc tggaactgaa a                                                 321
<210> 27
   <211> 369
   <212> DNA
   <213> artificial
<220>
   <223> 1022C3 antibody heavy chain variable domain (aat)
<400> 27
   caggttactc tgaaagagtc tggccctggg atattgcagc cctcccagac cctcagtctg        60
   acttgttctt tctctgggtt ttcactgaat acttctggta tgggtgtgag ctggattcgt       120
   cagccttcag gaaagggtct ggagtggctg gcacacattt actgggatga cgacaagcgc       180
   tataatccat ccctgaagag ccggctcaca atctccaagg atacctccag caaccaggtt       240
   ttcctcaaga tcaccagttt ggacactgca gatactgcca catactactg tgctcgaaga       300
   tctcactttg gtaactatta ctatgctatg gactactggg gtcaaggaac ctcagtcacc       360
   gtcacctca                                                               369
<210> 28
   <211> 369
   <212> DNA
   <213> artificial
<220>
   <223> 1022C3 antibody heavy chain variable domain (gac)
<400> 28
   caggttactc tgaaagagtc tggccctggg atattgcagc cctcccagac cctcagtctg        60
   acttgttctt tctctgggtt ttcactggac acttctggta tgggtgtgag ctggattcgt       120
   cagccttcag gaaagggtct ggagtggctg gcacacattt actgggatga cgacaagcgc       180
   tataatccat ccctgaagag ccggctcaca atctccaagg atacctccag caaccaggtt       240
   ttcctcaaga tcaccagttt ggacactgca gatactgcca catactactg tgctcgaaga       300
   tctcactttg gtaactatta ctatgctatg gactactggg gtcaaggaac ctcagtcacc       360
   gtcacctca                                                               369
<210> 29
   <211> 824
   <212> PRT
   <213> artificial
<220>
   <223> ADAM17
<400> 29
   Met Arg Gln Ser Leu Leu Phe Leu Thr Ser Val Val Pro Phe Val Leu
   1               5                   10                  15
   Ala Pro Arg Pro Pro Asp Asp Pro Gly Phe Gly Pro His Gln Arg Leu
               20                  25                  30
   Glu Lys Leu Asp Ser Leu Leu Ser Asp Tyr Asp Ile Leu Ser Leu Ser
           35                  40                  45
   Asn Ile Gln Gln His Ser Val Arg Lys Arg Asp Leu Gln Thr Ser Thr
       50                  55                  60
   His Val Glu Thr Leu Leu Thr Phe Ser Ala Leu Lys Arg His Phe Lys
   65                  70                  75                  80
   Leu Tyr Leu Thr Ser Ser Thr Glu Arg Phe Ser Gln Asn Phe Lys Val
                   85                  90                  95
   Val Val Val Asp Gly Lys Asn Glu Ser Glu Tyr Thr Val Lys Trp Gln
               100                 105                 110
   Asp Phe Phe Thr Gly His Val Val Gly Glu Pro Asp Ser Arg Val Leu
           115                 120                 125
   Ala His Ile Arg Asp Asp Asp Val Ile Ile Arg Ile Asn Thr Asp Gly
       130                 135                 140
   Ala Glu Tyr Asn Ile Glu Pro Leu Trp Arg Phe Val Asn Asp Thr Lys
   145                 150                 155                 160
   Asp Lys Arg Met Leu Val Tyr Lys Ser Glu Asp Ile Lys Asn Val Ser
                   165                 170                 175
   Arg Leu Gln Ser Pro Lys Val Cys Gly Tyr Leu Lys Val Asp Asn Glu
               180                 185                 190
   Glu Leu Leu Pro Lys Gly Leu Val Asp Arg Glu Pro Pro Glu Glu Leu
           195                 200                 205
   Val His Arg Val Lys Arg Arg Ala Asp Pro Asp Pro Met Lys Asn Thr
       210                 215                 220
   Cys Lys Leu Leu Val Val Ala Asp His Arg Phe Tyr Arg Tyr Met Gly
   225                 230                 235                 240
   Arg Gly Glu Glu Ser Thr Thr Thr Asn Tyr Leu Ile Glu Leu Ile Asp
                   245                 250                 255
   Arg Val Asp Asp Ile Tyr Arg Asn Thr Ser Trp Asp Asn Ala Gly Phe
               260                 265                 270
   Lys Gly Tyr Gly Ile Gln Ile Glu Gln Ile Arg Ile Leu Lys Ser Pro
           275                 280                 285
   Gln Glu Val Lys Pro Gly Glu Lys His Tyr Asn Met Ala Lys Ser Tyr
       290                 295                 300
   Pro Asn Glu Glu Lys Asp Ala Trp Asp Val Lys Met Leu Leu Glu Gln
   305                 310                 315                 320
   Phe Ser Phe Asp Ile Ala Glu Glu Ala Ser Lys Val Cys Leu Ala His
                   325                 330                 335
   Leu Phe Thr Tyr Gln Asp Phe Asp Met Gly Thr Leu Gly Leu Ala Tyr
               340                 345                 350
   Val Gly Ser Pro Arg Ala Asn Ser His Gly Gly Val Cys Pro Lys Ala
           355                 360                 365
   Tyr Tyr Ser Pro Val Gly Lys Lys Asn Ile Tyr Leu Asn Ser Gly Leu
       370                 375                 380
   Thr Ser Thr Lys Asn Tyr Gly Lys Thr Ile Leu Thr Lys Glu Ala Asp
   385                 390                 395                 400
   Leu Val Thr Thr His Glu Leu Gly His Asn Phe Gly Ala Glu His Asp
                   405                 410                 415
   Pro Asp Gly Leu Ala Glu Cys Ala Pro Asn Glu Asp Gln Gly Gly Lys
               420                 425                 430
   Tyr Val Met Tyr Pro Ile Ala Val Ser Gly Asp His Glu Asn Asn Lys
           435                 440                 445
   Met Phe Ser Asn Cys Ser Lys Gln Ser Ile Tyr Lys Thr Ile Glu Ser
       450                 455                 460
   Lys Ala Gln Glu Cys Phe Gln Glu Arg Ser Asn Lys Val Cys Gly Asn
   465                 470                 475                 480
   Ser Arg Val Asp Glu Gly Glu Glu Cys Asp Pro Gly Ile Met Tyr Leu
                   485                 490                 495
   Asn Asn Asp Thr Cys Cys Asn Ser Asp Cys Thr Leu Lys Glu Gly Val
               500                 505                 510
   Gln Cys Ser Asp Arg Asn Ser Pro Cys Cys Lys Asn Cys Gln Phe Glu
           515                 520                 525
   Thr Ala Gln Lys Lys Cys Gln Glu Ala Ile Asn Ala Thr Cys Lys Gly
       530                 535                 540
   Val Ser Tyr Cys Thr Gly Asn Ser Ser Glu Cys Pro Pro Pro Gly Asn
   545                 550                 555                 560
   Ala Glu Asp Asp Thr Val Cys Leu Asp Leu Gly Lys Cys Lys Asp Gly
                   565                 570                 575
   Lys Cys Ile Pro Phe Cys Glu Arg Glu Gln Gln Leu Glu Ser Cys Ala
               580                 585                 590
   Cys Asn Glu Thr Asp Asn Ser Cys Lys Val Cys Cys Arg Asp Leu Ser
           595                 600                 605
   Gly Arg Cys Val Pro Tyr Val Asp Ala Glu Gln Lys Asn Leu Phe Leu
       610                 615                 620
   Arg Lys Gly Lys Pro Cys Thr Val Gly Phe Cys Asp Met Asn Gly Lys
   625                 630                 635                 640
   Cys Glu Lys Arg Val Gln Asp Val Ile Glu Arg Phe Trp Asp Phe Ile
                   645                 650                 655
   Asp Gln Leu Ser Ile Asn Thr Phe Gly Lys Phe Leu Ala Asp Asn Ile
               660                 665                 670
   Val Gly Ser Val Leu Val Phe Ser Leu Ile Phe Trp Ile Pro Phe Ser
           675                 680                 685
   Ile Leu Val His Cys Val Asp Lys Lys Leu Asp Lys Gln Tyr Glu Ser
       690                 695                 700
   Leu Ser Leu Phe His Pro Ser Asn Val Glu Met Leu Ser Ser Met Asp
   705                 710                 715                 720
   Ser Ala Ser Val Arg Ile Ile Lys Pro Phe Pro Ala Pro Gln Thr Pro
                   725                 730                 735
   Gly Arg Leu Gln Pro Ala Pro Val Ile Pro Ser Ala Pro Ala Ala Pro
               740                 745                 750
   Lys Leu Asp His Gln Arg Met Asp Thr Ile Gln Glu Asp Pro Ser Thr
           755                 760                 765
   Asp Ser His Met Asp Glu Asp Gly Phe Glu Lys Asp Pro Phe Pro Asn
       770                 775                 780
   Ser Ser Thr Ala Ala Lys Ser Phe Glu Asp Leu Thr Asp His Pro Val
   785                 790                 795                 800
   Thr Arg Ser Glu Lys Ala Ala Ser Phe Lys Leu Gln Arg Gln Asn Arg
                   805                 810                 815
   Val Asp Ser Lys Glu Thr Glu Cys
               820
<210> 30
   <211> 260
   <212> PRT
   <213> artificial
<220>
   <223> Metalloprotease domain of ADAM17
<400> 30
   Arg Ala Asp Pro Asp Pro Met Lys Asn Thr Cys Lys Leu Leu Val Val
   1               5                   10                  15
   Ala Asp His Arg Phe Tyr Arg Tyr Met Gly Arg Gly Glu Glu Ser Thr
               20                  25                  30
   Thr Thr Asn Tyr Leu Ile Glu Leu Ile Asp Arg Val Asp Asp Ile Tyr
   35 40 45
   Arg Asn Thr Ser Trp Asp Asn Ala Gly Phe Lys Gly Tyr Gly Ile Gln
   50 55 60
   Ile Glu Gln Ile Arg Ile Leu Lys Ser Pro Gln Glu Val Lys Pro Gly
   65 70 75 80
   Glu Lys His Tyr Asn Met Ala Lys Ser Tyr Pro Asn Glu Glu Lys Asp
   85 90 95
   Ala Trp Asp Val Lys Met Leu Leu Glu Gln Phe Ser Phe Asp Ile Ala
   100 105 110
   Glu Glu Ala Ser Lys Val Cys Leu Ala His Leu Phe Thr Tyr Gln Asp
   115 120 125
   Phe Asp Met Gly Thr Leu Gly Leu Ala Tyr Val Gly Ser Pro Arg Ala
   130 135 140
   Asn Ser His Gly Gly Val Cys Pro Lys Ala Tyr Tyr Ser Pro Val Gly
   145 150 155 160
   Lys Lys Asn Ile Tyr Leu Asn Ser Gly Leu Thr Ser Thr Lys Asn Tyr
   165 170 175
   Gly Lys Thr Ile Leu Thr Lys Glu Ala Asp Leu Val Thr Thr His Glu
   180 185 190
   Leu Gly His Asn Phe Gly Ala Glu His Asp Pro Asp Gly Leu Ala Glu
   195 200 205
   Cys Ala Pro Asn Glu Asp Gln Gly Gly Lys Tyr Val Met Tyr Pro Ile
   210 215 220
   Ala Val Ser Gly Asp His Glu Asn Asn Lys Met Phe Ser Asn Cys Ser
   225 230 235 240
   Lys Gln Ser Ile Tyr Lys Thr Ile Glu Ser Lys Ala Gln Glu Cys Phe
   245 250 255
   Gln Glu Arg Ser
   260
<210> 31
   <211> 89
   <212> PRT
   <213> artificial
<220>
   <223> Disintegrin domain of ADAM17
<400> 31
   Asn Lys Val Cys Gly Asn Ser Arg Val Asp Glu Gly Glu Glu Cys Asp
   1               5                   10                  15
   Pro Gly Ile Met Tyr Leu Asn Asn Asp Thr Cys Cys Asn Ser Asp Cys
               20                  25                  30
   Thr Leu Lys Glu Gly Val Gln Cys Ser Asp Arg Asn Ser Pro Cys Cys
           35                  40                  45
   Lys Asn Cys Gln Phe Glu Thr Ala Gln Lys Lys Cys Gln Glu Ala Ile
       50                  55                  60
   Asn Ala Thr Cys Lys Gly Val Ser Tyr Cys Thr Gly Asn Ser Ser Glu
   65                  70                  75                  80
   Cys Pro Pro Pro Gly Asn Ala Glu Asp
                   85
<210> 32
   <211> 108
   <212> PRT
   <213> artificial
<220>
   <223> Membrane proximal domain of ADAM17
<400> 32
   Asp Thr Val Cys Leu Asp Leu Gly Lys Cys Lys Asp Gly Lys Cys Ile
   1               5                   10                  15
   Pro Phe Cys Glu Arg Glu Gln Gln Leu Glu Ser Cys Ala Cys Asn Glu
               20                  25                  30
   Thr Asp Asn Ser Cys Lys Val Cys Cys Arg Asp Leu Ser Gly Arg Cys
           35                  40                  45
   Val Pro Tyr Val Asp Ala Glu Gln Lys Asn Leu Phe Leu Arg Lys Gly
       50                  55                  60
   Lys Pro Cys Thr Val Gly Phe Cys Asp Met Asn Gly Lys Cys Glu Lys
   65                  70                  75                  80
   Arg Val Gln Asp Val Ile Glu Arg Phe Trp Asp Phe Ile Asp Gln Leu
                   85                  90                  95
   Ser Ile Asn Thr Phe Gly Lys Phe Leu Ala Asp Asn
               100                 105
<210> 33
   <211> 453
   <212> PRT
   <213> artificial
<220>
   <223> 1022C3 antibody heavy chain Chimeric (c) IgG1
<400> 33
   Gln Val Thr Leu Lys Glu Ser Gly Pro Gly Ile Leu Gln Pro Ser Gln
   1               5                   10                  15
   Thr Leu Ser Leu Thr Cys Ser Phe Ser Gly Phe Ser Leu Asn Thr Ser
               20                  25                  30
   Gly Met Gly Val Ser Trp Ile Arg Gln Pro Ser Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Ser Asn Gln Val
   65                  70                  75                  80
   Phe Leu Lys Ile Thr Ser Leu Asp Thr Ala Asp Thr Ala Thr Tyr Tyr
                   85                  90                  95
   Cys Ala Arg Arg Ser His Phe Gly Asn Tyr Tyr Tyr Ala Met Asp Tyr
               100                 105                 110
   Trp Gly Gln Gly Thr Ser Val Thr Val Thr Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Arg Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Glu Glu Met Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
   450
<210> 34
   <211> 452
   <212> PRT
   <213> artificial
<220>
   <223> 1022C3 antibody heavy chain Chimeric (c) IgG1 AlaAla
<400> 34
   Gln Val Thr Leu Lys Glu Ser Gly Pro Gly Ile Leu Gln Pro Ser Gln
   1 5 10 15
   Thr Leu Ser Leu Thr Cys Ser Phe Ser Gly Phe Ser Leu Asn Thr Ser
   20 25 30
   Gly Met Gly Val Ser Trp Ile Arg Gln Pro Ser Gly Lys Gly Leu Glu
   35 40 45
   Trp Leu Ala His Ile Tyr Trp Asp Asp Asp Lys Arg Tyr Asn Pro Ser
   50 55 60
   Leu Lys Ser Arg Leu Thr Ile Ser Lys Asp Thr Ser Ser Asn Gln Val
   65 70 75 80
   Phe Leu Lys Ile Thr Ser Leu Asp Thr Ala Asp Thr Ala Thr Tyr Tyr
   85 90 95
   Cys Ala Arg Arg Ser His Phe Gly Asn Tyr Tyr Tyr Ala Met Asp Tyr
   100 105 110
   Trp Gly Gln Gly Thr Ser Val Thr Val Thr Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Arg Val Glu Pro Lys
   210 215 220
   Ser Cys Asp Lys Thr His Thr Cys Pro Pro Cys Pro Ala Pro Glu Ala
   225 230 235 240
   Ala Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Glu Glu Met Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly
   450
<210> 35
   <211> 214
   <212> PRT
   <213> artificial
<220>
   <223> 1022C3 antibody light chain Chimeric (c) IgG1
<400> 35
   Asp Val Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Ser Tyr
               20                  25                  30
   Leu Asn Trp Tyr Gln Gln Lys Pro Asp Gly Thr Phe Lys Leu Leu Ile
           35                  40                  45
   Phe Tyr Thr Ser Arg Leu Gln Ser Gly Val Pro Ser Lys Tyr Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Ala Tyr Phe Cys Gln Gln Gly Lys Thr Leu Pro Leu
                   85                  90                  95
   Thr Phe Gly Ala Gly Thr Lys Leu Glu Leu Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 36
   <211> 1359
   <212> DNA
   <213> artificial
<220>
   <223> 1022C3 antibody heavy chain Chimeric (c)
<400> 36
   caggtgaccc tgaaggaaag cggacctggc attctgcagc catcacaaac actttcactg      60
   acatgcagtt tttccggttt ttctctgaat accagcggga tgggggtcag ctggatccgc     120
   cagcccagcg gtaagggact cgagtggctt gcccacatct attgggatga cgataagcgg     180
   tacaacccct ccctcaaaag cagactgact attagtaaag acacttctag taatcaggtg     240
   ttcttgaaga tcacctctct tgacacagcc gatactgcaa cttactactg cgctaggcga     300
   tcccatttcg gcaactatta ctatgccatg gattattggg gccagggcac atctgtcaca     360
   gttacctctg cttccaccaa gggcccttcc gtgttcccgc tagcccccag cagcaagagc     420
   accagcggcg gcacagccgc cctgggctgc ctggtgaagg actacttccc cgagcccgtg     480
   accgtgtcct ggaacagcgg agccctgacc tccggcgtgc acaccttccc cgccgtgctg     540
   cagagcagcg gcctgtacag cctgagcagc gtggtgaccg tgcccagcag cagcctgggc     600
   acccagacct acatctgtaa cgtgaaccac aagcccagca acaccaaggt ggacaagaga     660
   gtggagccca agagctgtga caagacccac acctgccccc cctgcccagc ccccgagctg     720
   ctgggcggac ccagcgtgtt cctgttcccc cccaagccca aggacaccct gatgatcagc     780
   agaacccccg aggtgacctg tgtggtggtg gacgtgtccc acgaggaccc agaggtgaag     840
   ttcaactggt acgtggacgg cgtggaggtg cacaacgcca agaccaagcc cagagaggag     900
   cagtacaaca gcacctacag ggtggtgtcc gtgctgaccg tgctgcacca ggactggctg     960
   aacggcaagg agtacaagtg taaggtgtcc aacaaggccc tgccagcccc aatcgaaaag    1020
   accatcagca aggccaaggg ccagccaaga gagccccagg tgtacaccct gccacccagc    1080
   agggaggaga tgaccaagaa ccaggtgtcc ctgacctgtc tggtgaaggg cttctaccca    1140
   agcgacatcg ccgtggagtg ggagagcaac ggccagcccg agaacaacta caagaccacc    1200
   cccccagtgc tggacagcga cggcagcttc ttcctgtaca gcaagctgac cgtggacaag    1260
   agcagatggc agcagggcaa cgtgttcagc tgctccgtga tgcacgaggc cctgcacaac    1320
   cactacaccc agaagagcct gagcctgtcc ccaggcaag                           1359
<210> 37
   <211> 1356
   <212> DNA
   <213> artificial
<220>
   <223> 1022C3 antibody heavy chain Chimeric (c) AlaAla
<400> 37
   caggtgaccc tgaaggaaag cggacctggc attctgcagc catcacaaac actttcactg      60
   acatgcagtt tttccggttt ttctctgaat accagcggga tgggggtcag ctggatccgc     120
   cagcccagcg gtaagggact cgagtggctt gcccacatct attgggatga cgataagcgg     180
   tacaacccct ccctcaaaag cagactgact attagtaaag acacttctag taatcaggtg     240
   ttcttgaaga tcacctctct tgacacagcc gatactgcaa cttactactg cgctaggcga     300
   tcccatttcg gcaactatta ctatgccatg gattattggg gccagggcac atctgtcaca     360
   gttacctctg cttccaccaa gggcccaagc gtgttccccc tggcccccag cagcaagagc     420
   accagcggcg gcacagccgc cctgggctgc ctggtgaagg actacttccc cgagcccgtg     480
   accgtgtcct ggaacagcgg agccctgacc tccggcgtgc acaccttccc cgccgtgctg     540
   cagagcagcg gcctgtacag cctgagcagc gtggtgaccg tgcccagcag cagcctgggc     600
   acccagacct acatctgtaa cgtgaaccac aagcccagca acaccaaggt ggacaagaga     660
   gtggagccca agagctgtga caagacccac acctgccccc cctgcccagc ccccgaggcg     720
   gcgggcggac ccagcgtgtt cctgttcccc cccaagccca aggacaccct gatgatcagc     780
   agaacccccg aggtgacctg tgtggtggtg gacgtgtccc acgaggaccc agaggtgaag     840
   ttcaactggt acgtggacgg cgtggaggtg cacaacgcca agaccaagcc cagagaggag     900
   cagtacaaca gcacctacag ggtggtgtcc gtgctgaccg tgctgcacca ggactggctg     960
   aacggcaagg agtacaagtg taaggtgtcc aacaaggccc tgccagcccc aatcgaaaag    1020
   accatcagca aggccaaggg ccagccaaga gagccccagg tgtacaccct gccacccagc    1080
   agggaggaga tgaccaagaa ccaggtgtcc ctgacctgtc tggtgaaggg cttctaccca    1140
   agcgacatcg ccgtggagtg ggagagcaac ggccagcccg agaacaacta caagaccacc    1200
   cccccagtgc tggacagcga cggcagcttc ttcctgtaca gcaagctgac cgtggacaag    1260
   agcagatggc agcagggcaa cgtgttcagc tgctccgtga tgcacgaggc cctgcacaac    1320
   cactacaccc agaagagcct gagcctgtcc ccaggc                              1356
<210> 38
   <211> 642
   <212> DNA
   <213> artificial
<220>
   <223> 1022C3 antibody light chain Chimeric (c)
<400> 38
   gacgtccaaa tgacacagac aacttcttcc ctgagcgctt ccttgggtga tcgagtgacc     60
   attagttgca gagcaagcca ggacatctct tcatacctca actggtatca gcaaaaacct    120
   gatggcacct tcaagctgct tattttctac accagtcggt tgcagtctgg cgttccatca    180
   aagtattccg gctctgggag tggcaccgac tactccctca ccatcagcaa tcttgagcaa    240
   gaggatatcg cagcctattt ctgccagcag ggaaagacac tccccctgac tttcggggct    300
   gggaccaagc tggaactgaa acgtacggtg gccgctccca gcgtgttcat cttcccccca    360
   agcgacgagc agctgaagag cggcaccgcc agcgtggtgt gtctgctgaa caacttctac    420
   cccagggagg ccaaggtgca gtggaaggtg gacaacgccc tgcagagcgg caacagccag    480
   gagagcgtca ccgagcagga cagcaaggac tccacctaca gcctgagcag caccctgacc    540
   ctgagcaagg ccgactacga gaagcacaag gtgtacgcct gtgaggtgac ccaccagggc    600
   ctgtccagcc ccgtgaccaa gagcttcaac aggggcgagt gc                       642